(19) Europäisches Patentamt
European Patent Office
Office européen des brevets

(11) Publication number : 0 650 970 A1

(12) **EUROPEAN PATENT APPLICATION**

(21) Application number : 94307926.9

(22) Date of filing : 27.10.94

(51) Int. Cl.[6] : C07F 7/08, C07H 23/00, // C08G77/60

(30) Priority : 27.10.93 US 143857

(43) Date of publication of application : 03.05.95 Bulletin 95/18

(84) Designated Contracting States : BE CH DE ES FR GB IT LI NL SE

(71) Applicant : Tidwell, Thomas T.
55 Colonel Danforth Trail
West Hill, Ontario M1C 1P8 (CA)

(71) Applicant : Zhao, Da-Chun
122 Brunswick Avenue
Toronto, Ontario M5S 2M2 (CA)

(71) Applicant : Allen, Annette D.
32 Woburn Avenue
Toronto, Ontario M5M 1K6 (CA)

(72) Inventor : Tidwell, Thomas T.
55 Colonel Danforth Trail
West Hill, Ontario M1C 1P8 (CA)
Inventor : Zhao, Da-Chun
122 Brunswick Avenue
Toronto, Ontario M5S 2M2 (CA)
Inventor : Allen, Annette D.
32 Woburn Avenue
Toronto, Ontario M5M 1K6 (CA)

(74) Representative : Sexton, Jane Helen et al
J.A. KEMP & CO.
14 South Square
Gray's Inn
London WC1R 5LX (GB)

(54) **Bisketene derivatives and process for production and use thereof.**

(57) The novel 1,2-bisketene of Formula I :

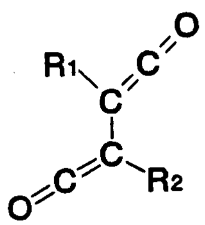

I

wherein :

$R^1$ is $SiR^3R^4R^5$ ;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen ; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group, is useful in e.g. preparing a succinate ester, or in reacting with a polyfunctional alcohol or ester to produce a polyester or polyamide.

EP 0 650 970 A1

## BACKGROUND OF THE INVENTION

## FIELD OF THE INVENTION

The present invention relates to novel bisketene derivatives and to a process for production thereof. The present invention also relates to the use of the bisketene derivatives.

## DESCRIPTION OF THE PRIOR ART

Ketenes are known and were first reported by Staudinger in Chem. Ber. **1905**, 38, 1735-1739, the contents of which are incorporated herein by reference. The simplest ketene has the formula

$$CH_2=C=O$$

and is also known as ethenone and carbomethene.

The discovery of bisketenes followed shortly thereafter and was reported by Diels et al. in Chem. Ber. **1906**, 39, 689-697, the contents of which are incorporated herein by reference. Specifically, Diels et al. disclosed preparation of carbon suboxide via dehydration of malonic acid according to the following reaction

$$CH_2(CO_2H)_2 + P_2O_5 \dashrightarrow O=C=C=C=O.$$

Staudinger et al. in Chem. Ber. **1908**, 41, 4461-4465 reported an alternate synthesis for carbon suboxide via a zinc catalyzed double debromination according to the following reaction

$$CBr_2(COBr)_2 + Zn \dashrightarrow O=C=C=C=O$$

The chemistry of carbon suboxide has been reviewed by Kappe et al. in Agnew. Chem. Int. Ed. Engl. **1974**, 13, 491-504, the contents of which are incorporated herein by reference.

Staudinger was, however, unsuccessful in his pursuit of a preparation for quinoketene (see Staudinger et al. in Chem. Ber. **1911**, 44, 1623-1633, the contents of which are incorporated herein by reference) and 1,2-bisketene (1,3-butadiene-1,4-dione, see Staudinger et al. in Helv. Chim. Acta **1923**, 6, 321-326, the contents of which are incorporated herein by reference).

Those bisketenes which have been synthesized, are quite reactive and, in some cases, unstable - see Hatchard et al. in J. Am. Chem. Soc. **1957**, 79, 6261-6263, Blomquist et al. in J. Am. Chem. Soc. **1957**, 79, 2021-2022, Staab et al. in Chem. Ber. **1968**, 101, 1457-1472, Boate et al. in J. Am. Chem. Soc. **1990**, 112, 8858-8863, Mosandl et al. in J. Org. Chem. **1993**, 58, 747-749, the contents of each of which are incorporated herein by reference.

The production of substituted 1,3-butadiene-1,4-diones via photolysis of cyclobutenediones is known. See Obata et al. in Chemical Communications **1971** 587-588 and Miller, et al, in J. Org. Chem **1993** 90-94, the contents of each of which are incorporated herein by reference. The parent 1,3-butadiene-1,4 dione has been generated by other routes (Kasai et al. in Chemistry Lett. **1978**, 217-218 and Maier, et al in Chem. Ber. **1982** 2192-2201, the contents of each of which are incorporated herein by reference). However, the product is a short-lived and unstable intermediate which has been observed only in an argon matrix. Typically, the bisketene reverts, via cyclization, to cyclobutenedione.

Several attempts have been made to prepare stable 1,2-bisketenes by conventional methods, however, the Applicant is not aware of the production of a stable 1,2-bisketene or a derivative thereof which is resistant to cyclization at ambient conditions. This in contrast to bisketene compounds where the ketenyl groups are separated from one another via an intervening aliphatic or aromatic group thereby stabilizing the compound - see, for example United States patent 3,002,024, the contents of which are incorporated herein by reference.

Thus, while numerous studies have been undertaken to prepare 1,2-bisketenes, to the Applicant's knowledge there is no known technique to form such compounds or derivatives thereof as stable materials which, for example permits isolation thereof.

## SUMMARY OF THE INVENTION

It is an object of the present invention to provide a novel 1,2-bisketene derivative.

It is another object of the present invention to provide a process for producing a 1,2-bisketene derivative.

It is yet another object of the present invention to provide a process for using a 1,2-bisketene derivative.

Accordingly, in one of its aspects, the present invention provides a 1,2-bisketene of Formula I

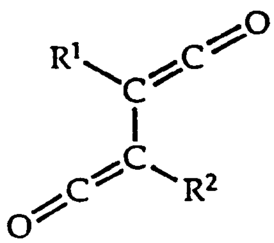

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a $C_{6-20}$ aryl group, a $C_{7-20}$ aralkyl group and a $C_{5-20}$ cycloalkyl group.

In another of its aspects the present invention provides a process for producing a 1,2-bisketene of Formula I

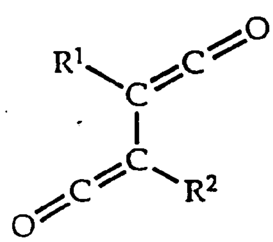

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a $C_{2-20}$ linear or branched alkyl group, a $C_{6-20}$ aryl group, a $C_{7-20}$ aralkyl group and a $C_{5-20}$ cycloalkyl group;

the process comprising the step of subjecting a compound of Formula II

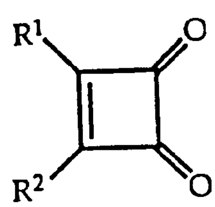

II

wherein $R^1$ and $R^2$ are as defined hereinbefore, to intramolecular ring opening to produce the 1,2-bisketene of Formula I.

In another of its aspects the present invention provides a process for forming a succinate ester, comprising the steps of:

forming a reaction mixture including a 1,2-bisketene of Formula I as defined hereinabove;

adding to the reaction mixture an alkoxide of an alcohol for reaction with the bisketene to form an enolate;

adding to the reaction mixture the alcohol for reaction with the enolate to form a monoketene;

adding to the reaction mixture the alkoxide to react with the monoketene to form an enolate; and

adding to the reaction mixture the alcohol for reaction with the enolate to form a succinate of formula III

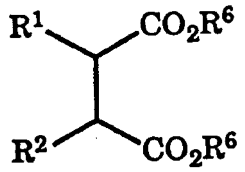

III

wherein $R^1$ and $R^2$ are defined as hereinabove and $R^6$ is selected from the group comprising methyl, a substituted or unsubstituted $C_{2-20}$ linear or branched alkyl group, a substituted or unsubstituted $C_{6-20}$ aryl group and a substituted or unsubstituted $C_{5-20}$ cycloalkyl group.

In still another of its aspects, the invention provides a succinate ester of formula III

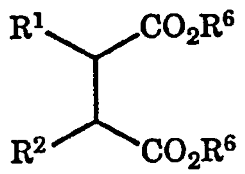

III

wherein $R^1$, $R^2$ and $R^6$ are as defined hereinabove.

In still another of its aspects, the invention provides a monoketene of Formula XXXV

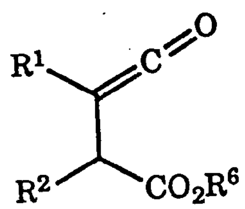

XXXV

wherein $R^1$ and $R^2$ and $R^6$ are as defined hereinabove.

## DETAILED DESCRIPTION OF THE PREFERRED EMBODIMENTS

Thus, an aspect of the present invention relates to the provision of a 1,2-bisketene compound of Formula I

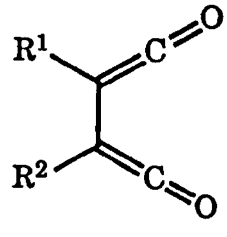

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a $C_{6-20}$ aryl group, a $C_{7-20}$ aralkyl group and a $C_{5-20}$ cycloalkyl group.

Preferred examples of $R^3$, $R^4$ and $R^5$ include $C_{2-6}$ linear or branched alkyl groups. Preferred examples of $R^1$ include trimethylsilyl [$(CH_3)_3Si$] and t-butyldimethylsilyl [$((CH_3)_3C)(CH_3)_2Si$], triisopropylsilyl and triphenylsilyl. Preferred examples of $R^2$ include trimethylsilyl, t-butyldimethylsilyl, phenyl, methyl, hydrogen, triisopropylsilyl and triphenylsilyl.

Preferably, both $R^1$ and $R^2$ are $SiR^3R^4R^5$, the same or different. More preferably, $R^1$ and $R^2$ are $SiR^3R^4R^5$, and are the same.

If desired, the substituents used in connection with the alkyl, aryl, aralkyl and cycloalkyl groups of $R^2$, $R^3$, $R^4$ and $R^5$ may themselves include a methyl, a $C_{2-20}$ linear or branched alkyl group, a $C_{6-20}$ aryl group, a $C_{5-20}$ cycloalkyl group and a halogen. In addition, the substituents may include alkene and alkyne groups and may contain one or more groups containing oxygen, nitrogen, sulphur, phosphorous and silicon. In addition, $R^2$, $R^3$, $R^4$ and $R^5$ may also include a substituted or unsubstituted $C_{2-20}$ linear or branched alkenyl group or a substituted or unsubstituted $C_{2-20}$ linear or branched alkynyl group.

To the applicant's knowledge, the compound of Formula I is believed to be the first stable and persistent 1,2-bisketene (1,3-butadiene-1,4-dione) and may be formed by several routes, including the thermochemical or photochemical reaction of the corresponding 2,3-bis(silyl-substituted)-cyclobut-3-ene-1,4-dione. These silyl stabilized 1,2-bisketene derivatives can be used as such or generated in situ thermally or photochemically, and are useful in reactions with, for example, polyfunctional alcohols to produce polyesters and polyfunctional amines to produce polyamides, the latter useful for producing nylons. It is contemplated that the present 1,2-bisketene derivatives may be used in the preparation of new specialty polymers of precisely controlled structure, as polymer coupling agents for polymers with hydroxyl or amino end-groups, and as cross-linking agents for a variety of polymers containing reactive hydroxyl groups such as carbohydrates, or polymers containing amino groups. Furthermore, when used as polymer coupling agents or crosslinking agents, the present 1,2-bisketene derivatives have the virtue of being reactive toward a wide variety of nucleophilic or electrophilic monomers and polymer end groups without the formation of byproducts. Other advantageous features of the present 1,2-bisketenes are that they are: (i) relatively inexpensive to produce, (ii) of low toxicity, and (iii) readily adaptable to existing polymer methodology.

A particular feature of the compound of Formula I is its ability to bond with polyfunctional alcohols via the two ketene groups. Specifically, the bonding rate of the first ketene group is significantly higher than that of the second, which enables the compound of Formula I to be used to form polymers in a controlled or even tailored fashion, by bonding one structure to the first ketene group and then another structure to the second ketene group.

$$\mathrm{I} \xrightarrow{HO(CH_2)_nOH} [O(CH_2)_nO_2CCHR^1CHR^2CO]_n$$

V

$$\mathrm{I} \xrightarrow{H_2N(CH_2)_nNH_2} [NH(CH_2)_nNHOCCHR^1CHR^2CO]_n$$

VI

Another feature of the compound of Formula I is its usefulness in the formation of polymers. Specifically, it has been discovered that the compound of Formula I may be polymerized with a polyfunctional alcohol and/or a polyfunctional amine to produce copolymers having $M_n$ of 2000 or more. The formation of the ester (according

to formula V) or amide bond (according to formula VI) occurs by an addition, and $H_2O$ is not formed as a by-product. This is an advantage for applications in which the polymerization must occur in a closed system.

$$I \xrightarrow{HO(CH_2)_nOH} O{=}C{=}CR^2CHR^1CO_2(CH_2)_nO_2CCHR^1CR^2{=}C{=}O$$

VII

It is believed that the compound of Formula I may be used directly as a polymer coupling reagent or it may be used to produce a polymer coupling agent according to formula VII) , for example, by acylation of a diol by two equivalents of the compound of Formula I to yield a novel bisketene with the ketene groups joined by an intervening diester unit. It is further believed that this intermediate may be purified and stored, or reacted directly with a new difunctional nucleophile to yield a polymer.

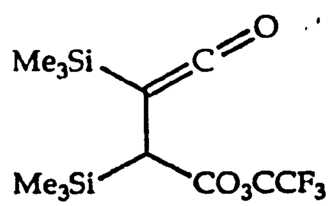

XXVI

Yet another feature of the compound of formula I is that it may be reacted with electrophilic reagents such as $CF_3CO_2H$ to form compounds (shown as formula XXVI) useful as difunctional acylating agents with two different acylating groups. These acylating agents should prove useful for selective reactions to produce polyesters, polyamides and other derivatives.

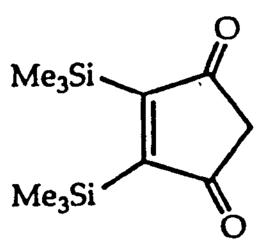

XXIX

Yet another feature of the compound of formula I is that it may be reacted with diazomethanes to form compounds, shown by formula XXIX, which are believed to have utility in the synthesis of steroids and prostaglandins.

The compound of Formula I may be produced by subjecting a compound of Formula II:

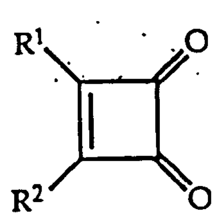

II

wherein $R^1$ and $R^2$ are as described above for compound of Formula I to a step of intramolecular ring opening.

Preferably, the step of intramolecular ring opening includes subjecting the compound of Formula II to electromagnetic radiation. Preferably, the electromagnetic radiation has a wavelength from about 200 to about 400 nanometers. More preferably, the radiation has a wavelength from 250 to 350 nanometers. This may be done in an inert solvent such as chloroform, hexane, or ether, preferably at a temperature of 50°C or below, and in the absence of oxygen.

Alternatively, the step of intramolecular ring opening may include subjecting the compound of Formula II to heat, preferably in a range from about 25°C to 250°C, and more preferably in a range of 60°C to 120°C. Above 250°C, the bisketenes may be unstable. The step of intramolecular ring opening may occur with the compound of formula II in a solid, melt, vapor phase or in solution. The compound may also be in a solid or liquid suspension.

A further feature of the compound of formula I is that two or more bisketene moieties thereof may be formed in a multiple-ketene structure, such as the tetraketene shown at

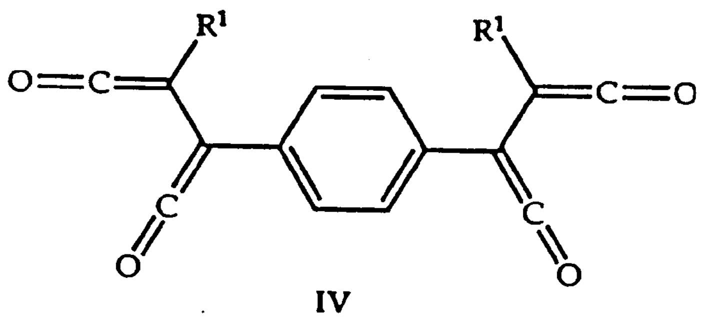

IV

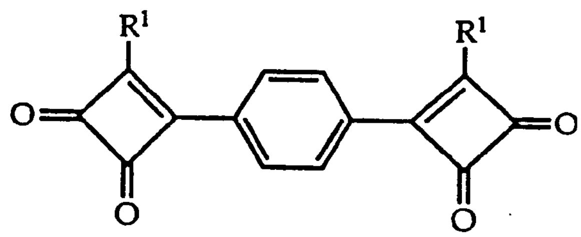

VIII

formula IV, with an intervening substituted or unsubstituted $C_{6-20}$ aryl group, a substituted or unsubstituted, linear or branched $C_{2-20}$ alkyl group or a substituted or unsubstituted $C_{2-20}$ cycloalkyl group. Preferably, the intervening group is a $C_{6-14}$ aryl group, a linear or branched $C_{2-6}$ alkyl group or a substituted or unsubstituted $C_{2-6}$ cycloalkyl group.

More preferable still are the substituents mentioned hereinabove for $R^2$, $R^3$, $R^4$ and $R^5$. Preferably, the multiple ketene structure is formed by way of a structure shown in formula VIII wherein two cyclobutenedione moieties are on opposite sides of an aryl ring. The step of intramolecular ring opening may then be carried out on the two cyclobutenedione moieties to yield the tetraketene of formula IV. As with their bisketene counterparts, the so-formed tetraketenes of formula IV are believed to have utility in polymer formation and cross-linking.

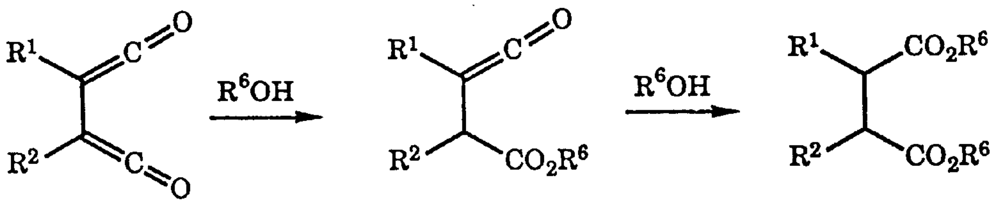


(1)

I XXXV III

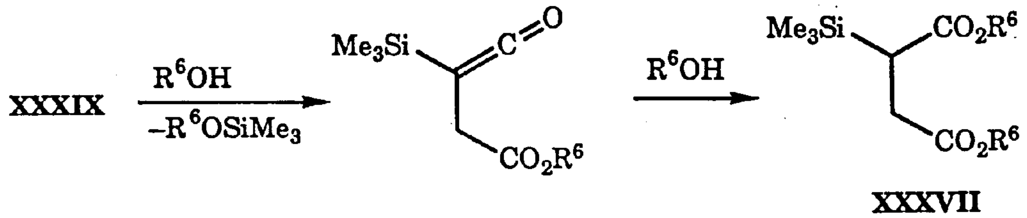


(1b)

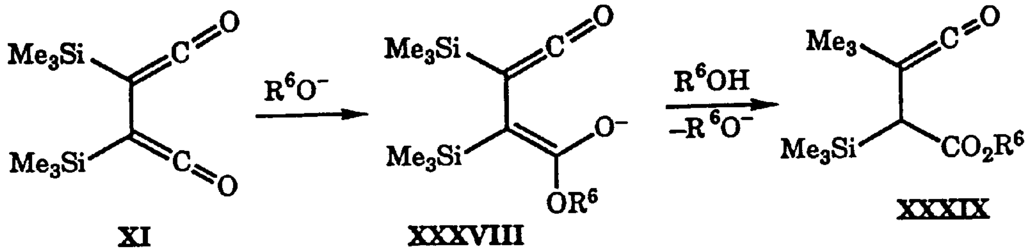


(2)

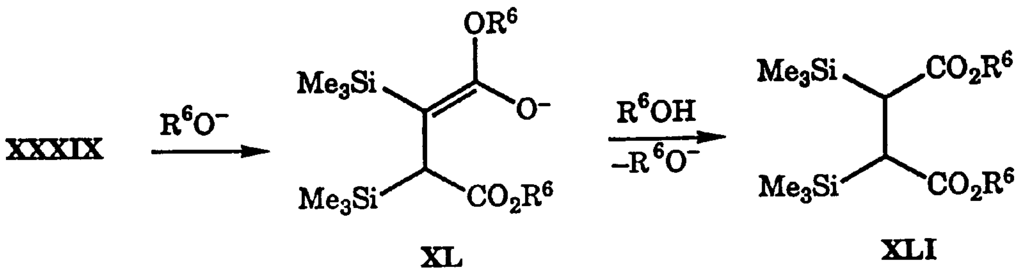


(3)

Referring to equation 1) another feature of the bisketene of formula I is that it may be reacted with alcohols to form monoketenyl esters, which themselves may be reacted with alcohols to form succinate esters of formula **III**. Depending on the choice of $R^1$, $R^2$ and $R^3$, the reaction to form the succinate ester may be more sluggish than the reaction to form the monoketenyl ester **XXXV** because of its greater stability and steric crowding compared to the bisketene **I**. This sluggish reaction to the succinate ester may also compete with a desilylation reaction to form mono(trialkylsilyl) succinate esters **XXXVII**.

Desilylation may be circumvented by the use of a basic catalyst such as the alkoxide of the alcohol. The alkoxide may be added separately or may be formed by reacting the alcohol with a catalyst such as an organo-sodium reagent $R^7Na$, an organo-lithium reagent $R^7Li$ or an organo-potassium reagent $R^7K$ thereby to form metal alkoxides $R^6ONa$, $R^6OLi$ and $R^6OK$ respectively, where $R^7$ is selected from the group comprising methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and a substituted or unsubstituted $C_{5-20}$ cycloalkyl group. Preferably, $R^7$ includes methyl, ethyl, n-butyl, t-butyl and iso-propyl. Alternatively, the alkoxide may be formed by reacting the alcohol with a metal hydride such as NaH, LiH or KH.

In the case of adding a catalyst, the alcohol reacts with the catalyst to form the alkoxide which, as demonstrated by equations 2 and 3, may then be reacted with the bisketene **XI** to form an enolate **XXXVIII**. The enolate **XXXVIII** may then be protonated by alcohol to generate a

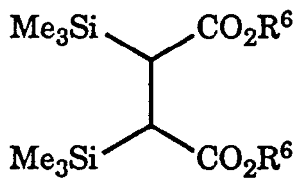

**XLI**

a $R^6$ = Me
b $R^6$ = Et
c $R^6$ = *i*-Pr
d $R^6$ = *t*-Bu
e $R^6$ = 4-Cl$C_6H_4$
f $R^6$ = Ph$CH_2$
g $R^6$ = *n*-Hx
h $R^6$ *n*-$C_{12}H_{25}$
i $R^6$ = 1,2:3,4-diisopropylidene galacto-6-pyranosyl

monoketene **XXXIX** and alkoxide, which may be reacted to form an enolate **XL** which may thereafter be protontated to yield a succinate **XLI**.

The basic catalyst may be used to form succinates **XLI(a-h)** which may occur in both stereoisomeric **dl-** and **meso-** forms, to be separated or differentiated by conventional methods, such as chromatography and X-ray crystallography, respectively.

In still another aspect of the present invention, there is provided a process to form a monoketene useful for the production of polymers, comprising the steps of:

forming a reaction mixture including a bisketene of formula I as defined hereinabove;

adding to the reaction mixture a compound having a first reactive group; and

subjecting the reaction mixture to conditions to cause the bisketene to react with the first reactive group to yield said monoketene.

In a preferred embodiment, the reaction mixture includes a compound having a second reactive group, further comprising the step of selecting conditions to cause the monoketene to react with the second reactive group.

In another preferred embodiment, the process further comprises the steps of:

removing the compound with the first reactive group from the reaction mixture; and

adding to the reaction mixture another compound including the second reactive group.

The compound may be an alcohol or an amine to lead to the formation of succinate esters and succinamides respectively. Preferred examples include those having methyl, ethyl, n-hexyl, t-butyl and iso-propyl groups.

As mentioned earlier, the bisketene may be used, in a variety of well established techniques, as an intermediate in the formation of a number of products. Its reactivity allows the bisketene to be used as a chain extender in the formation of longer acrylic and latex polymers and in place of isocyanates commonly used for this purpose. For example, the polymer may be placed in a reactor vessel along with the bisketene (in roughly equivalent proportions, depending on the molecular weights of the compounds and other factors) and held at a suitable temperature, such as 200°C, to form the longer polymers.

The bisketene may also be used as a cross-linking agent for a variety of polymers containing reactive hydroxyl groups such as carbohydrates, or polymers containing amino groups. This may be done using well established techniques. For example, the reaction may be carried out in a reaction vessel containing the bisketene and the polymer with or without the presence of a basic catalyst and at temperatures of about 200°C.

Embodiments of the present invention will be described with reference to the following Examples which are presented for illustrative purposes only and are not intended to limit the scope of the invention.

In the Examples, reference is made to activated zinc. There are several well known techniques to prepare activated zinc including the use of heat, ultrasonication and treatment with a copper salt to form a zinc-copper couple. These techniques, among others, are disclosed in the following references, all of which are incorporated by reference herein:

a) Danheiser et al., Organic Syntheses **1989**, 68, 32-40;
b) Danheiser et al.; Tetrahedron Letters **1983**, 23-26;
c) Deprés et al.; Organic Syntheses **1989**, 68, 41-48;
d) Mehta et al.; Synth. Commun. **1985**, 15, 991-1000;
e) Stenstrøm; Synth. Commun. **1992**, 22, 2801-2810; and

It should be noted that Stenstrøm discloses a technique for zinc activation comprising heating. This technique is believed to be the most effective of the activated zinc procedures disclosed.

Further reference will be made in the Examples to several various analytical and characterizing techniques used to identify various of the compounds obtained. These techniques are conventional and are within the purview of a person skilled in the art. Most of the techniques are discussed in the textbook "INTRODUCTION TO ORGANIC CHEMISTRY" by Heathcock, Kosower, Streitwieser (4th Edition), the contents of which are incorporated herein by reference.

EXAMPLE 1 - **2,3-Bis(trimethylsilyl)buta-1,3-diene-1,4-dione XI**

This Example describes the production of 2,3-bis(trimethylsilyl)buta-1,3-diene-1,4-dione of formula XI via 2,3-bis(trimethylsilyl)-4,4-dichlorocyclobut-2-enone of formula IX and 2,3-bis(trimethylsilyl)cyclobut-3-ene-1,4-dione of formula X.

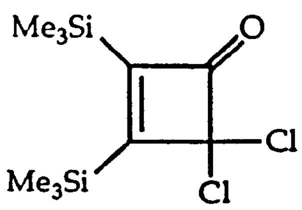

IX

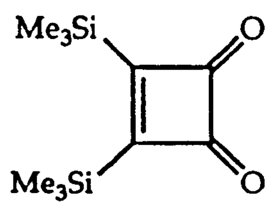

X

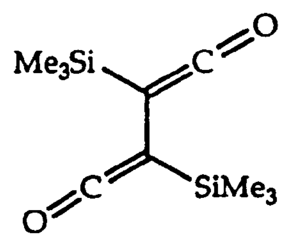

XI

**2,3-Bis(trimethylsilyl)-4,4-dichlorocyclobut-2-enone IX**. A 200 mL flask fitted with a dropping funnel and magnetic stirrer was heated and evacuated several times under argon. Bis(trimethylsilyl)acetylene (9.6 g, 56 mmol) was added followed by 50 mL ether and 10 g activated zinc. The activated zinc was produced using the technique described by Danheiser et al. referred hereinabove (Reference (a)). Through the dropping funnel was added trichloroacetyl chloride (21.8 g, 120 mmol) in 30 mL dimethoxyethane over a period of 1 hour at 25 °C, and the solution was stirred for 3 days. The reaction mixture was filtered through celite which was rinsed with pentene and the filtrate was washed with $H_2O$ and $NaHCO_3$, dried over $MgSO_4$, and evaporated to give 18.6 g of brownish oil. Radial chromatography (2% EtOAc in hexane, $R_f$ = 0.7) of a 100 mg portion of the crude product afforded 82 mg (88%). After recrystallization from pentane, the purified product had a melting point of 35.0-35.5°C. The $^1H$ NMR spectrum ($CDCl_3$) of the product had characteristic shifts (δ) at 0.278 and 0.421 ($Me_3Si$ groups). The $^{13}C$ NMR spectrum ($CDCl_3$) of the product had characteristic shifts (δ) at -1.64, - 1.06, 96.8, 169.1, 184.1, and 196.7. The IR spectrum (film) of the product had a characteristic absorption of 1776 $cm^1$ (C=O). The EIMS m/z of the product had characteristic peaks at 284, 282, 280 (20, $M^+$), 197, 195 (20), 174, 172 (25), 131, 129 (35), 73 (100, $Me_3Si^+$). The high resolution mass spectrum gave the value m/z of the molecular ion of 280.0283 (cf. calculated for $C_{10}H_{18}Cl_2OSi_2$ 280.0273).

**2,3-Bis(trimethylsilyl)cyclobut-3-ene-1,4-dione X**. Crude 2,3-bis(trimethylsilyl)-4,4-dichlorocyclobut-2-enone (0.95g, 3.36 mol) was added with vigorous stirring to concentrated $H_2SO_4$ (3 mL) in an Erlenmeyer flask in a water bath (55 °C). The solution was stirred for 12 minutes, and one quarter of the mixture was quenched with ice and extracted three times with ether. The combined ether extracts were washed with $NaHCO_3$ and $H_2O$, dried over $MgSO_4$, and evaporated. The product was subjected to chromatography on silica gel (7% EtOAc in hexane, $R_f$ = 0.5) to yield 2,3-bis(trimethylsilyl)cyclobut-3-ene-1,4-dione (0.14 g, 0.619 mmol, 73%) as a yellow solid. After recrystallization from pentane the product had a melting point of 50-52°C. The $^1H$ NMR spectrum ($CDCl_3$) of the product had a characteristic shift (δ) at 0.370 ($Me_3Si$). The $^{13}C$ NMR spectrum ($CDCl_3$) of the product had characteristic shifts (δ) at -1.57, 201.97, 217.09. The IR spectrum (solid film) of the product had a characteristic absorption at 1769 $cm^{-1}$. The UV spectrum (pentane) of the product had a $\lambda_{max}$ of 222 nm (ε 14,000), 268 (sh), and 354 nm (ε 37). The EIMS m/z of the product had characteristic peaks at 226 ($M^+$, 39), 170 ($M^+$-$C_2O_2$, 17), 155 ($M^+$-$CH_3$, $C_2O_2$, 100), 97 (18) and 73 (59). The high resolution mass spectrum gave the value m/z of the molecular ion of 226.0844 (cf. calculated for $C_{10}H_{18}O_2Si_2$ 226.0845).

**2,3-Bis(trimethylsilyl)buta-1,3-diene-1,4-dione XI (METHOD A)**. A sample of 2,3-bis(trimethylsilyl)cyclobut-3-ene-1,4-dione (20 mg) in $CDCl_3$ (0.6 mL) was degassed in a tube by three vacuum freeze-thaw cycles and sealed under $N_2$. The tube was then heated at 100°C for a period of 60 minutes to yield 2,3-bis(trimethylsilyl)buta-1,3-diene-1,4-dione as the only detectable product. The $^1H$ NMR spectrum ($CDCl_3$) of the product had a characteristic shift (δ) at 0.206 ($Me_3Si$). The $^{13}C$ NMR spectrum ($CDCl_3$) of the product had characteristic shifts (δ) at -0.94 ($Me_3Si$), 5.62 ($sp^2$ C) and 181.83 (sp C). The IR spectrum (film) of the product had a char-

acteristic absorption at 2084 $cm^1$ due to the ketene group. The UV spectrum (hexane) of the product had a $\lambda_{max}$ of 376 ($\varepsilon$ 110), 325 ($\varepsilon$ 250), 248 (sh, $\varepsilon$ 770). The EIMS m/z of the product had characteristic peaks at 226 ($M^+$, 28), 170 ($M^+$ $C_2O_2$, 14), 155 ($M^+$ $CH_3$, $C_2O_2$, 100), 97 (17) and 73 (54). The high resolution mass spectrum gave the value m/z of the molecular ion of 226.0836 (cf. calculated for $C_{10}H_{18}O_2Si_2$ 226.0845).

**2,3-Bis(trimethylsilyl)buta-1,3-diene-1,4-dione XI (METHOD B).** A sample of 2,3-bis(trimethysilyl)cyclobut-3-ene-1,4-dione was injected in hexane onto a 3 m X 1 cm OV-17 vapour phase chromatography column at 130 °C (injector temperature 250°C) and the product was collected with a retention time of 7 minutes. The product was characterized as 2,3-bis(trimethylsilyl)buta-1,3-diene-1,4-dione using the analytical techniques described in METHOD A.

**2,3-Bis(trimethylsilyl)buta-1,3-diene-1,4-dione XI (METHOD C).** A sample of 2,3-bis(trimethysilyl)cyclobut-3-ene-1,4-dione (18 mg) in degassed hexane (1.3 mL) was heated for 80 minutes at 100 °C. The product was characterized as 2,3-bis(trimethylsilyl)buta-1,3-diene-1,4-dione using the analytical techniques described in METHOD A.

**2,3-Bis(trimethylsilyl)buta-1,3-diene-1,4-dione XI (METHOD D).** A solution of 2,3-bis(trimethysilyl)cyclobut-3-ene-1,4-dione in $CDCl_3$ (0.02 M) was degassed and sealed in an NMR tube. The NMR tube was then irradiated at 5°C with electromagnetic radiation having a wavelength of 350 nm. After 12 minutes the $^1H$ NMR spectrum revealed the presence of 71% of 2,3-bis(trimethylsilyl)buta-1,3-diene-1,4-dione, 18% of residual 2,3-bis(trimethysilyl)cyclobut-3-ene-1,2-dione, and 11% bis(trimethylsilyl)acetylene ($\delta$ 0.170). After irradiation for 48 minutes, the $^1H$ NMR spectrum revealed the presence of 86% of 2,3-bis(trimethylsilyl)buta-1,3-diene-1,4-dione, 11% bis(trimethylsilyl)acetylene and 3% other unidentified compounds.

EXAMPLE 2 - **2,3-Bis(t-butyldimethylsilyl)buta-1,3-diene-1,4-dione XII**

This Example describes the production of 2,3-bis(t-butyldimethylsilyl)buta-1,3-diene-1,4-dione of formula XII via 2,3-bis(t-butyldimethylsilyl)-4,4-dichlorocyclobut-2-enone of formula XIII and 2,3-bis(t-butyldimethylsilyl)cyclobut-3-ene-1,4-dione of formula XIV.

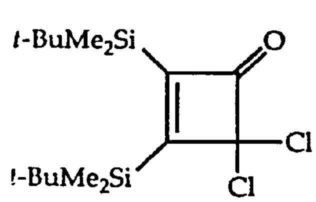

XIII

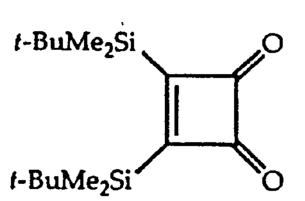

XIV

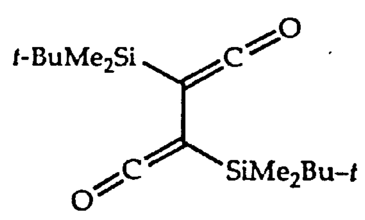

XII

**2,3-Bis(t-butyldimethylsilyl)-4,4-dichlorocyclobut-2-enone XIII.** The procedure described in Example 1 for the production of 2,3-bis(trimethylsilyl)-4,4-dichlorocyclobut-2-enone was repeated here except the acetylene compound used was bis(t-butyldimethylsilyl)acetylene (8.6 g, 33.8 mmol). Radial chromatography on silica gel yielded 10.4 g (28.5 mmol, 84%) product. The $^1H$ NMR spectrum ($CDCl_3$) of the product had characteristic shifts ($\delta$) at 0.261 (s, 6, $(CH_3)_2$), 0.410(s,6,$(CH_3)_2$), 0.933 (s, 9, t-butyl) and 1.033 (s, 9, t-butyl). The $^{13}C$ NMR spectrum ($CDCl_3$) of the product had characteristic shifts ($\delta$) at 4.98, -4.09, 16.91, 17.45, 26.85, 27.37, 96.46, 169.98, 184.77 and 194.74. The IR spectrum (film) of the product had a characteristic absorption of 1775 $cm^{-1}$ (carbonyl). The EIMS m/z of the product had characteristic peaks at 368, 366, 364 ($M^+$, 3, 12, 15), 279 (43), 195 (35), 151 (44), 73 ($Me_3Si^+$, 100) and 57 ($t\text{-}Bu^+$, 28). The high resolution mass spectrum gave the value m/z of the molecular ion of 364.1212 (cf. calculated for $C_{14}H_{30}Cl_2OSi_2$ 364.1214).

**2,3-Bis(t-butyldimethylsilyl)cyclobut-3-ene-1,4-dione XIV.** 2,3-Bis(t-butyldimethylsilyl)-4,4-dichlorocyclobut-2-enone (0.46 g, 1.25 mmol) was added with vigorous stirring to concentrated $H_2SO_4$ (1 mL) in Erlenmeyer flask in a water bath (54°C). The solution was stirred for 20 minutes. After work-up, the product was subjected to chromatography on silica gel (7% EtOAc in hexane, $R_f$ = 0.5) to yield 2,3-bis(t-butyldimethylsilyl)cyclobut-3-ene-1,4-dione (0.170 g, 0.55 mmol, 44%) as a yellow solid. After recrystallization from pentane the product had a melting point of 43.5-45.0°C. The $^1H$ NMR spectrum ($CDCl_3$) of the product had characteristic shifts ($\delta$) at 0.34 (s, 12, $2(CH_3)_2$) and 0.97 (s, 18, 2(t-butyl)). The $^{13}C$ NMR spectrum ($CDCl_3$) of the product had characteristic shifts at -5.13, 17.31, 26.74, 202.08 and 217.28. The IR spectrum (solid film) of the product had a characteristic absorption of 1766 $cm^{-1}$ (carbonyl). The UV spectrum (pentane) of the product had a $\lambda_{max}$

of 356 nm (e 27) and 224 nm (e 6,900). The EIMS m/z of the product had characteristic peaks at 310 ($M^+$, 100), 294 ($M^+$-O, 19), 197 (75), 171 (27), 155 (54, $M^+/2$) and 73 (73, $Me_3Si^+$). The high resolution mass spectrum gave the value m/z of the molecular ion of 310.1776 (cf. calculated for $C_{16}H_{30}O_2Si_2$ 310.1784).

**2,3-Bis(t-butyldimethylsilyl)buta-1,3-diene-1,4-dione XII (METHOD A).** A sample of 2,3-bis(t-butyldimethylsilyl)cyclobut-3-ene-1,4-dione in $CDCl_3$ was degassed in a tube by three vacuum freeze-thaw cycles and sealed under $N_2$. The tube was then heated at 100°C for a period of 60 minutes to yield 2,3-bis(t-butyldimethylsilyl)buta-1,3-diene-1,4-dione as the only detectable product. Thus, the $^1H$ NMR spectrum ($CDCl_3$) of the product had characteristic shifts (δ) at 0.172 (s, 12) and 0.956 (s, 18). The $^{13}C$ NMR spectrum ($CDCl_3$) of the product had characteristic shifts (δ) at -5.42, 4.27, 18.70, 26.38 and 182.24. The IR spectrum (film) of the product had a characteristic absorption of 2076 $cm^1$. The UV spectrum (hexane) of the product had a $\lambda_{max}$ of 400 (ε 88) and 326 (ε 88). The EIMS m/z of the product had characteristic peaks at 310 ($M^+$, 81), 197 ($MH^+$-$2C_4H_9$, 100), 171 (42), 155 (81) and 73 (78). The high resolution mass spectrum gave the value m/z of the molecular ion of 310.1796 (cf. calculated for $C_{16}H_{30}O_2Si_2$ 310.1784).

**2,3-Bis(t-butyldimethylsilyl)buta-1,3-diene-1,4-dione XII (METHOD B).** A solution of 2,3-bis(t-butyldimethysilyl)cyclobut-3-ene-1,4-dione in $CDCl_3$ was degassed and sealed in an NMR tube. The NMR tube was then irradiated at 5°C with electromagnetic radiation having a wavelength of 350 nm. After 48 minutes the $^1H$ NMR spectrum revealed the presence of 92% of 2,3-bis(t-butyldimethylsilyl)buta-1,3-diene-1,4-dione and 8% bis(t-butyldimethylsilyl)acetylene.

## EXAMPLE 3 - **2-Trimethylsilyl-3-phenyl-1,3-butadiene-1,4-dione XV**

This Example describes the production of 2-trimethylsilyl-3-phenyl-1,3-butadiene-1,4-dione of formula XV via 2-trimethylsilyl-3-phenyl-4,4-dichlorocyclobut-2-enone of formula XVI and 2-trimethylsilyl-3-phenylcyclobut-3-ene-1,4-dione of formula XVII.

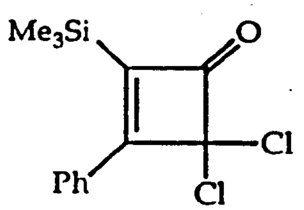

XVI

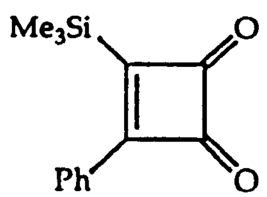

XVII

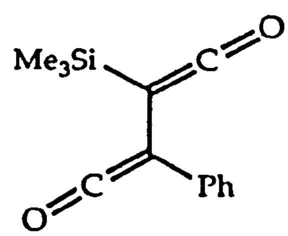

XV

**2-Trimethylsilyl-3-phenyl-4,4-dichlorocyclobut-2-enone XVI. (METHOD A)** This synthesis was carried out using an activated zinc-copper couple. The couple was produced using the technique described by Danheiser et al. referred hereinabove (Reference (a)) and **XVI** was obtained in a yield of 63% (melting point 72.5°C) after chromatography using 5/95 ethyl acetate on silica gel.

**2-Trimethylsilyl-3-phenyl-4,4-dichlorocyclobut-2-enone XVI. (METHOD B)** Trimethylsilylphenylacetylene was reacted with trichloroacetyl chloride in the presence of zinc dust with ultrasonication using the general procedure described by Mehta et al. referred hereinabove (Reference (d)). After purification, the $^1H$ NMR spectrum ($CDCl_3$) of the resulting product had characteristic shifts (δ) at 0.39 ($Me_3Si$), 7.53 (m, 3), and 7.92 (m, 2) (cf. Danheiser et al. (Reference (b) who reported characteristic shifts (δ) at 0.42, 7.61-7.63 and 7.98).

**2-Trimethylsilyl-3-phenylcyclobut-3-ene-1,4-dione XVII** A solution of 2-trimethylsilyl-3-phenyl-4,4-dichlorocyclobut-2-enone (1.0 g, 3.5 mmol), made according to Method D) above, in 5 mL anhydrous ether was added dropwise to 2.5 mL concentrated $H_2SO_4$ with stirring. The solution was heated to 95°C for 30 minutes, cooled with dry ice/acetone, and added to 100 mL of an ice and water mixture. The solution was extracted three times with 50 mL portions of ether. The combined ether extract was washed three times with 50 mL portions of $H_2O$, dried over $MgSO_4$ and concentrated on a rotary evaporator. The crude product (0.73 g) was purified by flash chromatography using 1:1 hexane:dichloromethane to yield 0.36 g (1.26 mmol, 35%) of a purified bright green solid having a melting point of 102.8°-103.2°C. The $^1H$ NMR spectrum ($CDCl_3$) of the product had characteristic shifts (δ) at 0.45 (s, 9, $(CH_3)_3Si$) and 7.50-8.00 (m, 5, $C_6H_5$). The $^{13}C$ NMR spectrum ($CDCl_3$) of the product had characteristic shifts (δ) at -1.4, 129.8, 130.0, 134.0, 157.2, 198.4, 199.9, 201.0 and 203.2. The IR spectrum ($CDCl_3$) of the product had characteristic absorptions ($cm^{-1}$) of 1776 (C=O), 1601 (C=C) and 1541 (m). The EIMS m/z of the product had characteristic peaks at 230 (10, $M^+$), 174 (39, $M^+$-2CO) and 159 (100, $M^+$-2CO, $CH_3$). The high resolution mass spectrum gave the value m/z of the molecular ion of 230.0758 (cf.

calculated for $C_{13}H_{14}SiO_2$ 230.0763). Elemental analysis of the product yielded C: 67.65%; H: 6.12%; and Si: 11.31% (cf. calculated for $C_{13}H_{14}SiO_2$ (230.34) C: 67.79%; H: 6.13%; and Si 12.19%).

**2-Trimethylsilyl-3-phenyl-1,3-butadiene-1,4-dione XV.** A solution of 2-trimethylsilyl-3-phenylcyclobut-3- ene-1,4-dione (0.0742 g, 0.322 mmol) in 0.5 mL $CDCl_3$ was sealed in an NMR tube under a $N_2$ atmosphere. The contents of the NMR tube were photolyzed 1½ hours with radiation having a wavelength of 350 nm. The $^1H$ NMR signals characteristic of 2-trimethylsilyl-3-phenylcyclobut-3-ene-1,4-dione decreased and new signals appeared corresponding to 87% isomerization to 2-trimethylsilyl-3-phenyl-1,3-butadiene-1,4-dione. Specifically, the $^1H$ NMR spectrum ($CDCl_3$) of the product had characteristic shifts (δ) at 0.23 (s, 9, $(CH_3)_3Si$) and 7.05-740 (m, 5, $C_6H_5$). The $^{13}C$ NMR spectrum ($CDCl_3$) of the product had characteristic shifts (δ) at -0.23, 7.9, 33.5, 124.4, 125.1, 129.4, 135.0, 178.8 and 202.2. The IR spectrum ($CDCl_3$) of the product had characteristic absorption ($cm^{-1}$) of 2076 (s) and 2042 (w). The UV spectrum ($CH_3CN$) of the product had a $\lambda_{max}$ of 257 nm.

EXAMPLE 4 - **2-Trimethylsilyl-3-methyl-1,3-butadiene-1,4-dione XVIII**

This Example describes the production of 2-trimethylsilyl-3-methyl-1,3-butadiene-1,4-dione of formula XVIII via 2-trimethylsilyl-3-methyl-4,4-dichlorocyclobut-2-enone and 2 trimethylsilyl 3-methylcyclobut-3-ene-1,4-dione.

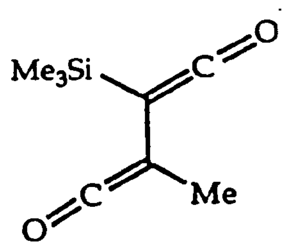

XVIII

To a 100 mL 3-necked round bottom flask containing Zn dust, 1-(trimethylsilyl)-1-propyne (1.01 g, 9.0 mmol) and 50 mL of anhydrous ether were added. Through a dropping funnel, trichloroacetyl chloride (3.03 g, 16.6 mmol) in 15 mL of anhydrous ether was added dropwise over a period of 30 minutes with stirring and sonication. The stirring was continued for 5 hours. The reaction mixture was filtered through Celite which was rinsed with ether under vacuum suction. The resulting brown filtrate was washed successively with distilled water, saturated $NaHCO_3$ solution and distilled water. The brown organic layer was dried over anhydrous $MgSO_4$, filtered by gravity and the solvent was evaporated to yield crude 2-trimethylsilyl-3-methyl-4,4-dichlorocyclobut-2-enone. The analytical data of this compound agreed with that previously reported by Danheiser et al. (Reference (b)).

The crude 2-trimethylsilyl-3-methyl-4,4-dichlorocyclobut-2-enone hydrolyzed with $H_2SO_4$ using the procedure described in Example 3 to produce 2-trimethylsilyl-3-methylcyclobut-3-ene-1,4-dione, which was purified by chromatography to give a yellow liquid. The $^1H$ product had characteristic shifts (δ) at -2.27, 13.20, 200.08, 201.18, 207.11, and 208.70. The IR spectrum (film) of the product had a characteristic absorption ($cm^{-1}$) of 1711 $cm^{-1}$(C=O). The UV spectrum (hexane) of the product had a $\lambda_{max}$ of 222 nm. The EIMS m/z of the product had characteristic peaks at 168 ($M^+$), 140 ($M^+$ -CO), and 112 ($M^+$ - $C_2O_2$). The high resolution mass spectrum gave the value m/z of the molecular ion of 168.0615 (cf. calculated for $C_8H_{12}OSi$ 168.0607).

The 2-trimethylsilyl-3-methylcyclobut-3-ene-1,4-dione was subjected to photolysis using the procedure described in Example 3 to produce 2-trimethylsilyl-3-methyl-1,3-butadiene-1,4-dione. The $^1H$ NMR spectrum ($CDCl_3$) had characteristic shifts (δ) at 0.20 ($Me_3Si$) and 1.74 (Me).

EXAMPLE 5 - **Diethyl-2-trimethylsilylsuccinate XIX**

This Example describes the production of diethyl 2-trimethylsilylsuccinate of formula XIX via 2,3-bis(trimethylsilyl)-buta-1,3-diene-1,4-dione of formula XI and 3 carboethoxy-2,3-bis(trimethylsilyl)prop-1-ene-1-one of formula XX.

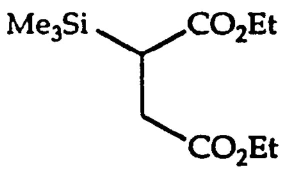

XIX

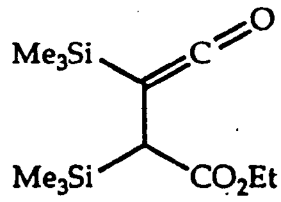

XX

**3-Carboethoxy-2,3-bis(trimethylsilyl)prop-1-ene-1-one XX.** The bisketene 2,3-bis(trimethylsilyl)buta-1,3-diene-1,4-dione, produced in Example 1 (0.020 g, 0.088 mol) was dissolved in 0.7 mL cold EtOH and kept at 0°-5°C for a period of 20 minutes. The ethanol was evaporated while the solution warmed to room temperature, and the only observed product was 3-carboethoxy-2,3-bis(trimethylsilyl)prop-1-ene-1-one. Specifically, the product was confirmed by an $^1$H NMR spectrum ($CDCl_3$) having characteristic shifts (δ) at 0.143 (s, 9, $(CH_3)_3Si$), 0.151 (s, 9, $(CH_3)_3Si$), 1.266 (t, 3, J=7.12 Hz, $CH_3$), 1.962 (s, 1, CH) and 4.155 (q, 2, J=7.12 Hz, $CH_2$). The $^{13}$C NMR spectrum ($CDCl_3$) of the product had characteristic shifts (δ) at -2.27, -0.91, 10.82, 14.38, 30.20, 60.80, 175.05 and 181.11. The IR spectrum ($CDCl_3$) of the product had characteristic absorptions ($cm^{-1}$) of 2093 (sh), 2085 (s) and 1711 (s). The UV spectrum ($CH_3CN$/ethanol, 5/1) had a $\lambda_{max}$ of 205 nm. The EIMS m/z of the product had characteristic peaks at 272 ($M^+$, 28) 243 (38), 147 (35) and 73 (100, $TMS^+$). The high resolution mass spectrum gave the value m/z of the molecular ion of 272.1264 (cf. calculated for $C_{12}H_{24}O_3Si_2$ 272.1263).

**Diethyl 2-trimethylsilylsuccinate XIX.** 3 Carboethoxy-2,3-bis(trimethylsilyl)prop-1-ene-1-one was refluxed in ethanol for 16 hours. The solvent was then evaporated evaporation of the solvent, and examination by $^1$H NMR indicated the presence of 25% diethyl succinate and 75% diethyl 2-trimethylsilylsuccinate. The latter product was separated by vapor phase chromatography. The $^1$H NMR spectrum ($CDCl_3$) of the separated product had characteristic shifts (δ) shifts at 0.100 (s, 9, $(CH_3)_3Si$), 1.251 (t, 6, J=7.1 Hz, $CH_3$), 2.34 (dd, 1, J=16.7, 3.3 Hz), 2.48 (dd, 1, J=11.4, 3.3 Hz), 2.8 (dd, 1, J=16.7, 11.4 Hz) and 4.13 (q, 4, J=7.13 Hz). The $^{13}$C NMR spectrum ($CDCl_3$) of the separated product had characteristic shifts (δ) -2.86, 14.1, 14.4, 31.3, 32.9, 60.0, 60.6, 172.9 and 174.5. The IR spectrum (film) of the separated product at characteristic absorption ($cm^{-1}$) of 1758, 1733, 1722 and 1715. The EIMS m/z of the separated product had characteristic peaks at 246 ($M^+$, 1), 201 ($M^+$-OEt, 39), 173 ($M^+$ $Me_3Si$, 73), 129 (41), 103 (51) and 73 ($Me_3Si^+$, 95). The high resolution mass spectrum gave the value m/z of the molecular ion of 231.1048 (cf. calculated for $C_{10}H_{19}O_4Si$ is 231.1053).

EXAMPLE 6 - **Dimethyl 2,3-bis(t-butyldimethylsilyl)succinate XXI**

This Example describes the production of dimethyl 2,3-bis(t-butyldimethylsilyl)succinate of formula XXI via 2,3-bis(t-butyldimethylsilyl)buta-1,3-diene-1,4-dione of formula XII and 3 carbomethoxy-2,3-bis(t-butyldimethylsilyl)prop-1-ene-1-one of formula XXII.

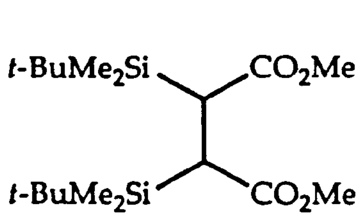

XXI

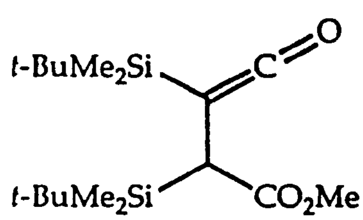

XXII

**3 Carbomethoxy-2,3-bis(t-butyldimethylsilyl)prop-1-ene-1-one XXII.** 2,3-Bis(t-butyldimethylsilyl)buta-1,3-diene-1,4-dione was prepared using the general procedure described in Example 1 (METHOD A) and was thereafter reacted with 0.5 mL of methanol for 5 hours at 7°C followed by evaporation of the solvent to yield 3 carbomethoxy-2,3-bis(t-butyldimethylsilyl)prop-1-ene-1-one. The $^1$H NMR spectrum ($CDCl_3$) of the product had characteristics shifts (δ) at 0.078 (s, 3), 0.111 (s, 3), 0.115 (s, 3), 0.137 (s, 3), 0.892 (s, 9), 0.938 (s, 9), 2.123 (s, 1) and 3.677 (s, 3). The $^{13}$C NMR spectrum ($CDCl_3$) of the product had characteristic shifts

(δ) at -6.89, -5.94, -5.60, 9.78 ($C_b$), 17.77 19.04, 26.32, 26.56, 51.73, 176.09 and 181.27. The IR spectrum ($CDCl_3$) of the product had characteristic absorptions ($cm^{-1}$) of 2085 (s) and 1720 (s). The EIMS m/z of the product had characteristic peaks at 342 ($M^+$, 23), 257 (52), 196 (40) and 73 (100). The high resolution mass spectrum gave the value m/z of the molecular ion of 342.2054 (cf. calculated for $C_{17}H_{34}O_3Si_2$ 342.2047).

**Dimethyl 2,3-bis(t-butyldimethylsilyl)succinate XXI.** 3 Carbomethoxy-2,3-bis(t-butyldimethylsilyl) prop-1-ene-1-one (99 mg, 0.319 mmol) was reacted with methanol at 90°C for 100 minutes. The methanol was then removed by evaporation and purification of the product by radial chromatography on silica gel yielded dimethyl 2,3-bis(t-butyldimethylsilyl)succinate (100 mg, 84%) having a melting point of 78.5-80.5°C. The $^1H$ HMR spectrum ($CDCl_3$) of the product had characteristic shifts (δ) at -0.011 (s, 6), 0.145 (s, 6), 0.924 (s, 18), 2.277 (s, 2) and 3.635 (s, 6). The $^{13}C$ NMR spectrum ($CDCl_3$) of the product had characteristic shifts (δ) at -6.14, -6.04, 17.87, 26.85, 30.60, 50.99 and 174.85. The IR (film) spectrum of the product had a characteristic shift at 1733 $cm^{-1}$ (s). The EIMS m/z of the product had characteristic peaks at 374 ($M^+$, 3), 317 ($M^+$ t-butyl, 15), 289 (16), 143 (39), 389 (41) and 73 (100). The high resolution mass spectrum gave the value m/z of the molecular ion of 374.2299 (cf. calculated for $C_{18}H_{38}O_4Si$ 374.2309).

EXAMPLE 7 - **Dimethyl 2-trimethylsilyl-3-phenylsuccinates XXIII**

This Example describes the production of dimethyl 2-trimethylsilyl-3-phenylsuccinates of formula XXIII via 2-trimethylsilyl-3-phenyl-1,3-butadiene-1,4-dione of formula XV and 2-trimethylsilyl-3 carbomethoxy-3-phenylprop-1-ene-1-one of formula XXIV.

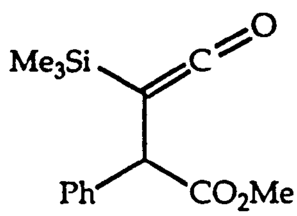

XXIV

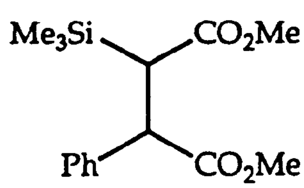

XXIII

**2-Trimethylsilyl-3-carbomethoxy-3-phenylprop-1-ene-1-one XXIV.** The solution of 2-trimethylsilyl-3-phenyl-1,3-butadiene-1,4-dione prepared according to the procedure described in Example 3 above was cooled to -78 °C and $CH_3OH$ (20 μL, 0.50 mmol) was added with shaking. The solution was allowed to warm to room temperature over 10 minutes and the solvent was removed on a rotary evaporator. The resulting residue was dissolved in $CDCl_3$ again, and the solution was subjected to photolysis and methanolysis using the procedures described in Example 7 above. Purification by VPC on an OV-17 column at 200°C yield pure 2-trimethylsilyl-3 carbomethoxy-3-phenylprop-1-ene-1-one (57.3 mg, 0.218 mmol, 68%): The $^1H$ NMR spectrum ($CDCl_3$) of the product had characteristic shifts (δ) at 0.00 (s, 9, $(CH_3)_3Si$), 3.68 (s, 3, $CH_3$), 4.00 (s, 1, CH) and 7.20-7.50 (m, 5, $C_6H_5$). The $^{13}C$ NMR spectrum ($CDCl_3$) of the product had characteristic shifts (δ) at -0.6, 8.5, 47.7, 53.3, 128.3, 128.4, 129.1, 139.5, 173.9 and 194.1. The IR ($CDCl_3$) spectrum of the product had a characteristic shift at 2093 $cm^{-1}$ (C=O). The EIMS m/z of the product had characteristic peaks at 262 (2, $M^+$), 247 (35, $M^+$-$CH_3$), 219 (11, $M^+$ $CH_3$, CO), 203 (73, $M^+$-$CO_2CH_3$) and 73 (100, $Me_3Si^+$). The high resolution mass spectrum gave the value m/z of the molecular ion of 262.1009 (cf. calculated for $C_{14}H_{18}O_3Si$ 262.1025).

**Dimethyl 2-trimethylsilyl-3-phenylsuccinates XXIII.** 2-Trimethylsilyl-3 carbomethoxy-3-phenylprop-1-ene-1-one (9.8 mg, 0.037 mmol) in 0.5 mL $CDCl_3$ containing $CH_3OH$ (20 μL, 16 mg, 0.50 mmol) in an NMR tube was allowed to react for 10 hours at room temperature. The starting material was completely converted to a 2:1 mixture of the threo/erythro stereoisomers of dimethyl 2-trimethylsilyl-3-phenylsuccinate, as analyzed by $^1H$ NMR. After separation of the stereoisomers using radial chromatography, the products were analyzed and following results were obtained confirming the identity of each product.

Threo-(dimethyl 2-trimethvlyilyl-3-phenylsuccinate):

Melting point: 59°-61°C;
Shifts (δ) in $^1H$ NMR spectrum ($CDCl_3$):

0.13 (s, 9, $(CH_3)_3Si$), 3.10 (d, 1, J=12.4 Hz, CHTMS), 3.38 and 3.62 (each s, 3, $OCH_3$), 4.02 (d, 1, J=12.4 Hz, $CHC_6C_5$) and 7.24-7.34 (s, 5, Ph);

Shifts (δ) in $^{13}C$ NMR spectrum ($CDCl_3$):
-2.091, 41.053, 49.723, 50.887, 52.147, 127.354, 127.863, 128.427, 138.980, 173.209 and 173.263;
Absorptions ($cm^{-1}$) in IR spectrum ($CDCl_3$):
1719 and 1731 $cm^{-1}$ (C=O);
Peaks in EIMS m/z (rel intensity):
294 (1, $M^+$), 279 (6, $M^+-CH_3$), 235 (24, $M^+-CO_2CH_3$), 131 (100, $M^+$-TMS, $CO_2CH_3$, $OCH_3$), 73 (26, $TMS^+$); and
High resolution mass spectrum m/z:
Found 294.1275/$M^+$ requires 294.1287.

Erythro-(dimethyl 2-trimethylsilyl-3-phenylsuccinate):

Shifts (δ) in $^1H$ NMR spectrum ($CDCl_3$):
-0.21 (s, 9, $(CH_3)_3Si$), 2.96 (d, 1, J=12.4, CHTMS), 3.60 and 3.69 (each s, 9, $OCH_3$), 4.09 (d, 1, J=12.4 Hz, $CHC_6H_5$), 7.31 (br s, 5, $C_6H_5$);
Shifts (δ) in $^{13}C$ NMR spectrum ($CDCl_3$):
-2.140, 41.022, 50.337, 51.306, 52.314, 127.952, 128.718, 128.811, 136.962, 174.412 and 175.026;
Absorptions ($cm^{-1}$) in IR spectrum ($CDCl_3$):
1710 and 1736 cm 1 (C=O);
Peaks in EIMS m/z (rel intensity):
294 (3, $M^+$), 279 (18, $M^+-CH_3$), 263 (8, $M^+-CH_3O$), 235 (47, $M^+-CO_2CH_3$), 131 (100, $M^+$-TMS, $CO_2CH_3$, $OCH_3$), 73 (38, $MTS^+$); and
High resolution mass spectrum m/z:
Found 294.1267/$M^+$ requires 294.1287.

EXAMPLE 8 - **2,3-Bis(trimethylsilyl)-4-trifluoroacetoxybut-1-ene-1,4-dione XXVI**

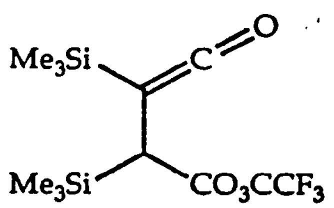

XXVI

To the 2,3-bis(trimethlysilyl)buta-1,3-diene-1,4-dione (see Example 1 above for preparation, 28.0 mg, 0.124 mmol) in 0.5 mL $CDCl_3$ in an NMR tube at room temperature was added $CF_3CO_2H$ (14.1 mg, 0.124 mmol). Analysis of the product confirmed the formation of 2,3-bis(trimethylsilyl)-4-trifluoroacetoxybut-1-ene-1,4-dione as the sole product. The $^1H$ NMR spectrum ($CDCl_3$) of the product had characteristic shifts (δ) 0.186 (s, 9), 0.241 (s, 9) and 2.214 (s, 1). The $^{13}C$ NMR spectrum ($CDCl_3$) of the product had characteristic shifts (δ) at -2.31, -1.14, 9.93, 32.14, 113.80 (q, 1, $J_{13C-19F}$ = 286.2 Hz), 167.22, 174.16 (q, 1, $J_{13C-19F}$ = 63.3 Hz, $COCF_3$) and 179.46. The IR spectrum ($CDCl_3$) of the product had characteristic absorptions ($cm^{-1}$) of 2095, 1837 and 1767.

EXAMPLE 9 - **1,2-Bis(trimethylsilyl)cyclopent-1-ene-3,5-dione XXIX**

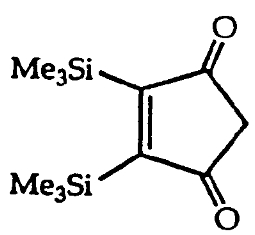

XXIX

Approximately 63.2 mg of 2,3-bis(trimethylsilyl)buta-1,3-diene-1,4-dione (see Example 1 for preparation, 0.279 mmol) was placed in a 25 mL three-necked round bottomed flask along with 10 mL of anhydrous diethyl ether. A reflux condenser was attached and the solution was placed under $N_2$ flow and stirred magnetically. Approximately 31.9 mg of $(CH_3)_3SiCHN_2$, prepared according to the procedure described in Seyferth et al., J. Organomet. Chem. **1972**, 44, 279-290 (the contents of which are incorporated herein by reference), was added dropwise via syringe and stirring was continued overnight. The ether solution was washed with $H_2O$ (2 x 10 mL), dried over anhydrous magnesium sulfate and concentrated by rotary evaporation to yield a yellow liquid/brownish-red solid mixture. This mixture was dissolved in 10% ethyl acetate/90% hexane and rotary chromatographed, using a 10% ethyl acetate/90% hexane solvent system to yield yellow crystals of pure 1,2-bis(trimethylsilyl)cyclopent-1-ene-3,5-dione having a melting point of 29°-33°C. Thus, the $^1H$ NMR spectrum ($CDCl_3$) of the product had characteristic shifts (δ) at 0.344 (s, H) and 2.756 (s, $(CH_3)_3Si$). The $^{13}C$ NMR spectrum ($CDCl_3$) of the product had characteristic shifts (δ) at 0.514 ($(CH_3)_3Si$), 41.184 ($CH_2$), 175.481 (C=C) and 205.799 (CO). The UV spectrum (hexane) of the product had a $\lambda_{max}$ of 434 nm, $\varepsilon_{434nm}$=49.5 $cm^{-1}M^{-1}$, shoulder peaks at 250 nm. The EIMS m/z of the product had characteristic peaks at 240 ($M^+$, 24), 225 ($M^+$ $CH_3$), 197 (51, $M^+$-CO, $CH_3$), 155 (100, $M^+$-$CH_2CO$, CO, $CH_3$) and 73 (74). The high resolution mass spectrum gave the value m/z of the molecular ion of 240.0994 (cf. calculated for $C_{11}H_{20}O_2Si_2$ 240.1002).

EXAMPLE 10 - **1,4-Bis[2'-(3'-trimethylsilylbuta-1',4'-dioxo-1',3'-dienyl)]-benzene XXX**

This Example describes the production of the tetraketene 1,4-bis[2'-(3'-trimethylsilylbuta-1',4'-dioxo-1',3'-dienyl)]-benzene of formula XXX via 1,4-bis(trimethylsilylethynyl)benzene of formula XXXI, 1,4-bis(4',4'-dichloro-2'-trimethylsilyl-3'-oxo-1'-cyclobutenyl)benzene of formula XXXII and 1,4-bis(2'-trimethylsilyl-3',4'-dioxo-1'-cyclobutenyl)benzene of formula XXXIII.

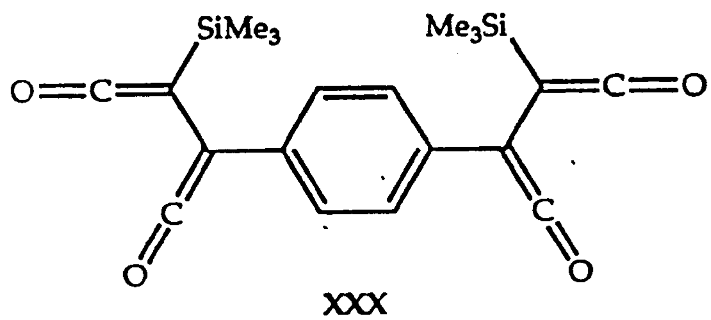

XXX

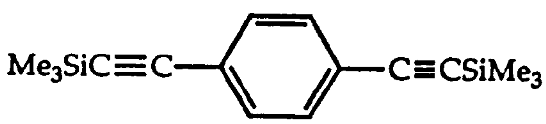

XXXI

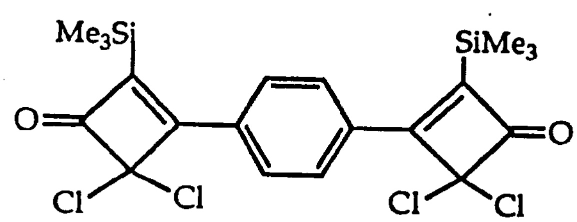

XXXII

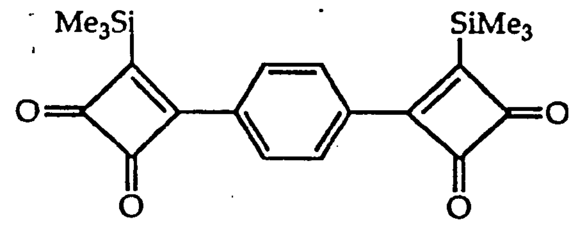

XXXIII

**1,4-bis(trimethylsilylethynyl)benzene XXXI**. The following chemicals were charged into a 500 mL three-necked flask equipped with a dry ice condenser: 250 mL triethylamine, diiodobenzene (20.0 g, 0.061 mol), triphenylphosphine (0.65 g, 2.48 mmol), cuprous iodide (0.24 g, 1.26 mmol), and trimethylsilylacetylene (14.7 g, 0.15 mol). Thereafter, palladium(II)acetate (0.32g, 1.42 mmol) was added and the reaction mixture was stirred at room temperature for 4 hours under nitrogen and then the solvent was removed by rotary evaporator. The residue was recrystallized from hexanes to yield colourless crystals of 1,4-bis(trimethylsilylethynyl)benzene (14.5g, yield 88%). This compound is known see Nast et al, J. Organomet. Chem. 1979, 182, 197-202, the contents of which are encorporated herein by reference.

**1,4-Bis[1'-(4',4'-dichloro-2'-trimethylsilyl-3'-oxo-1'-cyclobutenyl)]benzene XXXII**. To a dry, 250 mL round bottom flask containing 1,4-bis(trimethylsilylethynyl)benzene under nitrogen was added zinc dust (20.0g, 7.41 mmol) and 40 ml anhydrous ether. The flask was then partially submerged in a sonicator water bath and trichloroacetyl chloride (32.6 g, 0.18 mol) dissolved in 75 mL anhydrous ether was dropwise added for 2 hours with sonication which was continued for another 2 hours. The solution was filtered through celite, 10 mL dimethoxyethane was added to precipitate zinc chloride, and the solution was filtered several more times. The solution was then evaporated and the residue was separated by flash chromatography using 10% acetyl acetate and 90% hexane. Further purification by recrystallization from ethyl acetate and hexane was then carried out to yield light yellow crystals of 1,4-bis(4',4'-dichloro-2'-trimethylsilyl-3'-oxo-1'-cyclobutenyl)benzene (1.2 g, yield 33%) having a melting point 181.0°-182.0°C. The $^1H$ NMR spectrum ($CDCl_3$) of the product had characteristic shifts (δ) at 0.49 (s, 18H, $(CH_3)_3Si$) and 8.80 (s, 4H, $C_6H_4$). The $^{13}C$ NMR spectrum ($CDCl_3$) of the product had characteristic shifts (δ) at -1.55, 25.6, 130.4, 132.0, 157.6, 178.4 and 182.3. The IR spectrum ($CDCl_3$) of the product had characteristic absorptions ($cm^{-1}$) of 1799 (s, C=O), 620 (C=C) and 1550. The EIMS m/z of the product had characteristic peaks at 492 ($M^+$,27), 449 ($M^+$-Cl), $CH_3$ (15), 149 ($M^+$-4Cl, 2CO, 2TMS, 38). The high resolution mass spectrum gave the value m/z of the molecular ion of 489.9924 (cf. calculated for $C_{20}H_{22}Cl_4O_2Si_2$ 489.9912).

**1,4-Bis[1'-(2'-trimethylsilyl-3',4'-dioxo-1'-cyclobutenyl)benzene XXXIII**. To a stirred round bottom flask charged with 0.4 g 1,4-bis(4',4'-dichloro-2'-trimethylsilyl-3'-oxo-1'-cyclobutenyl)benzene, was added dropwise 15 mL 96.7% sulfuric acid. The reaction mixture was maintained at a temperature of 84°-86°C and stirred for 2 hours. The reaction mixture was slowly added to 100 mL of an ice-water mixture and extracted there times with 20 mL portions of ether. The ether extracts were combined, washed three times with 30 mL portions of water, dried over $MgSO_4$ and concentrated by rotary evaporation. The crude product (0.25 g) was recrystallized from dichloromethane and ethyl acetate to yield 1,4-bis(2'-trimethylsilyl-3',4'-dioxo-1'-cyclobutenyl)benzene (0.08g, 25%) as a yellow solid. The $^1H$ NMR spectrum ($CDCl_3$) of the product had characteristic shifts (δ) at 0.50 (s, 18, $(CH_3)_3Si$) and 8.1 (s, 4, $C_6,H_5$). The $^{13}C$ NMR spectrum ($CDCl_3$) of the product had characteristic shifts (δ) at -1.8, 129.8, 132.7, 197.0, 197.5, 199.9 and 205.6. The IR spectrum ($CDCl_3$) of the product had characteristic absorptions ($cm^{-1}$) of 1732 (s, C=O), 1774 (w, C=O) and 1488 (m, C=C). The EIMS m/z of the product had characteristic peaks at 382 ($M^+$, 25), 326($M^+$-2CO, 28) and 293($M^+$-TMS, O,33). The high resolution mass spectrum gave the value m/z of the molecular ion of 382.1047 (cf. calculated for $C_{20}H_{22}O_4Si_2$ 382.1057).

**1,4-Bis[2'-(3'-trimethylsilylbuta-1',4'-dioxo-1',3'-dienyl)]-benzene XXX**. Photolysis at 350 nm of 1,4-bis(2'-trimethylsilyl-3',4'-dioxo-1'-cyclobutenyl)benzene (0.05g in 0.5 ml $CDCl_3$) resulted in conversion to 1,4-bis[2'-(3'-trimethylsilylbuta-1',4'-dioxo-1',3'-dienyl)]-benzene. The $^1H$ NMR spectrum ($CDCl_3$) of the product had characteristic shifts (δ) at 0.20 (s,18,$Me_3Si$) and 7.05 (S, 4, Ar). The $^{13}C$ NMR spectrum ($CDCl_3$) of the product had characteristic shifts (δ) at -0.84, 6.88, 32.77, 124.22, 127.7, 178.02 and 203.05. The IR spectrum ($CDCl_3$) of the product had a characteristic absorption of 2086 $cm^1$ (C=C=O).

## EXAMPLE 11 -Succinates

This example describes the production of succinates via **2,3-bis(trimethylsilyl)buta-1,3-diene-1,4-dione XI** in the presence of an n-butyllithium catalyst.

In each of the examples to follow, a small amount of n-butyllithium was added to a pentane solution of the alcohol (Aldrich, 2M solution in pentane, the molar ratio of n-BuLi to the alcohol was <1) followed by addition of a pentane solution of bisketene **XI** (the molar ratio of alcohol to bisketene was >2:1 or 2:1). After an appropriate interval the reaction mixture was diluted with pentane and water was added. The organic layer was separated, dried, and evaporated to give the corresponding succinate as solids or oils as a mixture of dl and meso isomers, as indicated by NMR analysis.

### dl- and meso Dimethyl succinates XLI(a)

To a heterogeneous mixture of methanol (0.037 mL, 0.9 mmol) and 0.05 mL of a 2 M pentane solution of n-BuLi (0.1 mmol) in pentane (0.1 mL), was added about 30 mg of bisketene **XI.** One hour later the reaction progress was monitored by withdrawing an aliquot of the reaction mixture. A mixture of the **XLI(a)** diastereomers was observed. The $^1H$ NMR spectrum ($CDCl_3$) of the product had characteristic shifts ($\delta$) at 0.06 and 0.13 (each s, $Me_3Si$ of meso- and dl- **XLI(a)**, respectively), 2.64 and 2.30 (CHCO of meso- and dl- **XLI(a)**, respectively), 3.62 and 3.64 (each s, $CH_3$ groups of meso- and dl-**XLI(a)**, respectively). The IR spectrum of the product had characteristic absorptions of 1715 (s), 1435 (s); The EIMS m/z of the product had characteristic peaks at 290 ($M^+$, 2), 275 ($M^+$ -$CH_3$, 12), 247 (14), 186 (26), 171 (48), 73 (100); The high resolution mass spectrum gave the value m/z of the molecular ion of 290.1397 (calculated for $C_{12}H_{26}O_4Si_2$ 290.1370).

### dl- and meso Diethyl succinates XLI(b)

To 1.2 mL of pentane containing anhydrous ethanol (0.047 mL, 36.8 mg, 0.8 mmol) and 0.05 mL of a 2M pentane solution of n-BuLi (0.1 mmol), was added the bisketene **XI** (34 mg, 0.15 mmol). The resulting yellow solution was kept for 20 min at room temperature, and part of the solution was withdrawn and analyzed as a mixture of the **XLI(b)** diastereomers. The $^1H$ NMR spectrum ($CDCl_3$) of the product had characteristic shifts ($\delta$) at 0.08 and 0.14 (each s, $Me_3Si$ of meso- and dl-**XLI(b)**, respectively), 1.26 and 1.25 (each t, $CH_3$ groups of dl- and meso-**XLI(b)** isomers), 2.61 and 2.27 (CHCO of meso- and dl-**XLI(b)**, respectively) and 4.10 (m, $CH_2$ groups of dl- and meso-**XLI(b)**). The IR spectrum of the product had characteristic absorptions of 1713 (s), 1583 (s), 1035 (m). The EIMS m/z of the product had characteristic peaks at 318 ($M^+$, 2), 303 ($M^+$ -$CH_3$, 6) 289 ($M^+$ -$C_2H_5$, 49), 171 (77), 73 (100). The high resolution mass spectrum gave the value m/z of the molecular ion of 318.1687 (calculated for $C_{14}H_{30}O_4Si_2$: 318.1683).

### dl- and meso Di-i-propyl succinates XLI(c).

To 1.2 mL of pentane containing isopropanol (0.061 mL, 48 mg, 0.80 mmol) was added 0.04 mL of a 2M pentane solution of n-BuLi (0.08 mmol). To the resulting solution was added a pentane solution of the bisketene **XI** (20 mg) at room temperature and the resulting homogenous mixture turned brown from yellow in a few min. After 20 min part of the mixture was withdrawn and the solvent evaporated with a stream of nitrogen and the residue was taken up with 0.6 mL of $CDCl_3$ and analyzed as a mixture of the **XLI(c)** diastereomers with the $^1H$ NMR ($CDCl_3$) of the product having characteristic shifts ($\delta$) at 0.09 and 0.14 (each s, $Me_3Si$ meso- and dl-**XLI(c)**), 2.56 and 2.19 (CHCO meso- and dl-**XLI(c)**, respectively), 4.95 (m, $CHMe_2$ of dl- and meso-**XLI(c)**). The IR spectrum (film) of the product had characteristic absorptions of 1710 (s), 1104 (s). The EIMS m/z of the product had characteristic peaks at 346 ($M^+$, 2),303 ($M^+$, -$C_3H_7$, 6), 261 (54), 245 (57), 171 (61), 147 (48), 73 (100). The high resolution mass spectrum gave the value m/z of the molecular ion of 346.1983 (calculated for $C_{16}H_{34}O_4Si_2$: 346.1995).

### dl and meso Di-t-butyl succinates XLI(d)

To 1.2 mL of pentane containing t-butanol (0.075 mL, 0.81 mmol) was added 0.03 mL of a 2M pentane solution of n-BuLi (0.06 mmol). To the resulting homogeneous solution was added a pentane solution of the bisketene **XI** (27 mg, 0.12 mmol) at room temperature. The color of the solution turned to brown from yellow within a few minutes, and the solution was left for 80 min. Work up as above gave 40.6 mg of the **XLI(d)** diastereomers as a colorless solid: The $^1H$ NMR spectrum ($CDCl_3$) of the product had characteristic shifts ($\delta$) at 0.15 and 0.12 (each s, $Me_3Si$ dl and meso-**XLI(d)**, respectively), 1.45 (s, t-Bu, dl and meso-**XLI(d)**, accidental

degeneracy), 2.08 and 2.46 (each s, CHCO, dl- and meso-**XLI(d)**, respectively). The $^{13}C$ NMR spectrum ($CDCl_3$) of the product had characteristic shifts (δ) at -0.43, 28.4, 35.5, 79.6, 173.6 (dl); -0.69, 28.5, 36.4, 80.1, 174.2 (meso): The IR spectrum (Nujol mull) of the product had a characteristic absorption of 1719 cm $^{1}$. The EIMS m/z of the product had characteristic peaks at 262 ($M^+$ -2$C_4H_8$, 26), 245 (25), 217 (17), 172 (47), 147 (100); CIMS (i-$C_4H_{10}$) m/z 375 ($M^+$ +1, 5), 319 ($M^+$ -$C_4H_7$, 13), 263 ($M^+$ +1 -2$C_4H_8$, 100). The high resolution mass spectrum gave the value m/z of the ion ($M^+$ -2 $C_4H_9$) of 262.1050 (calculated for $C_{10}H_{22}O_4Si_2$, 262.1057).

**dl and meso Di-4-chlorophenyl 2,3-bis(trimethylsilyl)succinate XLI(e)**

To 4-chlorophenol (104 mg, 0.811 mmol, 6.1 eq) in 1.5 mL of pentane was added 20 μL (0.032 mmol, 4.0 mol % based on the phenol) of a 1.6 M solution of n-BuLi in hexanes. After 15 min 30mg bisketene **XI** dissolved in 1.5 mL of pentane was added to the phenol in one portion. After 2 hours all of the solids had dissolved, and the reaction mixture was poured into pentane/water. The phases were separated and the organic layer was washed three times with water and once with brine. Drying over $MgSO_4$ and evaporation of the solvent yielded 50 mg of a colorless solid, which was shown by $^1H$ NMR spectrum analysis to be a 5:1 mixture of meso- and dl-**XLI(e)**, respectively. Recrystallization from pentane yielded pure meso-**XLI(e)** (29.1 mg, 45%): colorless solid; with a melting point of 111.6-112.3 °C. The IR spectrum of the product had characteristic absorptions of 2959, 1743, 1497, 1272, 1202, 1082, 850, 512 cm $^{1}$. The $^1H$ NMR spectrum ($CDCl_3$) of the product had characteristic shifts (δ) at 0.253 (s, 18H), 2.951 (s, 2H), 7.08 (m, 4H), 7.35 (m, 4H). The $^{13}C$ NMR spectrum ($CDCl_3$) of the product had characteristic shifts (δ) at - 0.91, 35.73, 122.75, 129.45, 130.99, 149.08, 172.70. The EIMS m/z of the product had characteristic peaks at 482 (9), 439 (21), 355 (23), 327 (100), 282 (16), 267 (15), 239 (19), 200 (41), 185 (46), 127 (80), 99 (18), 73 (87). The high resolution mass spectrum gave the value m/z of the molecular ion of 482.0905 (calculated for $C_{22}H_{82}Cl_2O_4$, 482.0903). Pure dl-XLI(e) was not isolated: The $^1H$ NMR spectrum ($CDCl_3$) of the product had characteristic shifts (δ) at 0.28 (s, 18H), 2.61 (s, 2H), 6.76 (m, 4H), 7.18 (m, 4H). The structure of meso-XLI was established by X-ray crystallography.

**dl- and meso Dibenzyl succinates XLI(f)**

To a 2-necked 10 mL round bottom flask under Ar fitted with two septa and a magnetic stirrer was added benzyl alcohol (50 μL, 0.483 mmol) in 1 mL diethyl ether followed by 1.6 M n-BuLi (250 μL, 0.400 mmol) solution in hexane with stirring. Bisketene **XI** (46.7 mg, 0.207 mmol) in 1 mL diethyl ether was then added dropwise with stirring and the solution was stirred for 17 h at 25 °C. Then 3 mL of diethyl ether was added and the mixture was washed with water, dried over anhydrous $MgSO_4$, and evaporated to give 0.0413 g of pale yellow crystals shown by $^1H$ NMR spectrum analysis to be a 3.5:1 mixture of the **XLI(f)** diastereomers which were separated by radial chromatography (4% EtOAc in hexane) as colorless crystals.

**(meso-)XLI(f):** The $^1H$ NMR spectrum ($CDCl_3$) of the product had characteristic shifts (δ) at 0.020 (s, 18, $Me_3Si$), 2.685 (s, 2, CHCO), 4.946 and 5.109 (each d, 2, $CH_2$), 7.4 (m, 10, $C_6H_5$). The $^{13}C$ NMR spectrum ($CDCl_3$) of the product had characteristic shifts (δ) at - 1.62, 35.45, 66.22, 128.24, 128.49, 128.74, 135.69, 174.40. The IR spectrum ($CCl_4$) of the product had characteristic absorptions of 1713 cm $^{1}$; The CIMS m/z of the product had characteristic peaks at 443 ($MH^+$), 425, 397, 377, 335 ($M^+$ - $C_7H_7O$), 307 ($M^+$ - $C_7H_7O$ - CO), 279 ($M^+$ -$C_7H_7O$ - 2CO), 245, 91 ($C_7H_7$), 73 ($Me_3Si$). The high resolution mass spectrum gave the value m/z of the ion ($M^+$ - $CH_3$) of 427.1777 (calculated for $C_{23}H_{31}O_4Si_2$ 427.1761).

**(dl-)XLI(f):** The $^1H$ NMR spectrum ($CDCl_3$) of the product had characteristic shifts (δ) at 0.088 (s, 18, $Me_3Si$), 2.367 (s, 2, CHCO), 4.955 and 5.076 (each d, 2, $CH_2$), 7.337 (m, 10, $C_6H_5$). The IR spectrum ($CCl_4$) of the product had characteristic absorptions of 1726 cm $^{1}$. The EIMS m/z of the product had characteristic peaks at 427 ($M^+$ - $CH_3$), 397, 367, 351 ($M^+$ - $C_7H_7$), 321, 307 ($M^+$ - $C_7H_7O$ - CO), 277, 233, 91 ($C_7H_7$), 73 ($Me_3Si$).

**dl- and meso-Di-n-hexyl succinates XLI(g)**

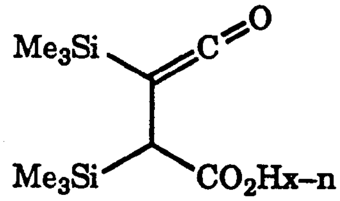

**XLV**

To 2 mL hexane containing n-hexanol (52 μL,42.3 mg, 0.415 mmol) in a 10 mL flask under argon was added 1.6 M n-BuLi (130 μL, 0.208 mmol) in hexane with stirring. Bisketene **XI** (46.2 mg, 0.204 mmol) in 1 mL hexane was added dropwise and the solution was stirred for 30 min at 25 °C. Then 5 mL hexane was added and the mixture was washed twice with $H_2O$, dried over anhydrous $MgSO_4$, and evaporated to give 0.0449 g of yellow liquid, which was shown by $^1H$ NMR analysis to be a 5/3/3.4 mixture of the monoketene **XLV** and **meso** and **dl XLI(g)** which were separated by radial chromatography (4% EtOAc in hexane):

**XLV:** The $^1H$ NMR spectrum ($CDCl_3$) of the product had characteristic shifts (δ) at 0.14 (s, 9, $Me_3Si$), 0.15 (s, 9, $Me_3Si$), 0.9 (m, 3, $CH_3$), 1.3 (m, 6, $(CH_2)_3$), 1.6 (m, 2, $OCH_2CH_2$), 1.96 (s, 1, $Me_3SiCH$), 4.07 (m, 2, $CH_2O$). The $^{13}C$ NMR spectrum ($CDCl_3$) of the product had characteristic shifts (δ) at - 2.25, -0.91, 10.9, 14.0, 22.5, 25.6, 28.7, 30.2, 31.4, 65.1, 175.1, 181.1. The IR spectrum (film) of the product had characteristic absorptions of 2087 cm $^{1}$ (C=C=O), 1721 cm $^{1}$ ($CO_2Me$). The EIMS m/z of the product had characteristic peaks at 328 ($M^+$, 15), 243 ($M^+$ - $C_6H_{13}$, 40), 154 ($M^+$ - $C_6H_{13}O$, $Me_3Si$, 32), 147 (36), 73 ($Me_3Si^+$, 100). The high resolution mass spectrum gave the value m/z of the molecular ion of 328.1888 (calculated for $C_{16}H_{32}O3Si_2$ 328.1890).

**meso XLI(g):** The $^1H$ NMR spectrum ($CDCl_3$) of the product had characteristic shifts (δ) at 0.07 (s, 18, $Me_3Si$), 0.9 (m, 6, $CH_3$), 1.3 (m, 12, $(CH_2)_3$), 1.6 (m, 4, $OCH_2\underline{CH_2}$), 2.61 (s, 2, $Me_3SiCH$), 3.98 (m, 4, $OCH_2$). The $^{13}C$ NMR spectrum ($CDCl_3$) of the product had characteristic shifts (δ) at -1.62, 13.97, 22.53, 25.74, 28.57, 31.41, 35.42, 64.29, 174.80. The IR spectrum of the product had a characteristic absorption of ($CCl_4$) 1712 cm $^{1}$. The EIMS m/z of the product had characteristic peaks at 430 ($M^+$, 7), 415 ($M^+$ - $CH_3$, 5), 402 ($M^+$ - CO), 387 ($M^+$ - $CH_3$, CO, 14), 345 ($M^+$ - $C_6H_{13}$, 14), 329 ($M^+$ - $C_6H_{13}O$, 7), 301 ($M^+$ - $C_6H_{13}O$, CO, 24), 245 ($M^+$ - $CH_3$, $2C_6H_{13}$, 76), 201 (32), 171 ($M^+$ - $2C_6H_{13}O$ and $Me_2Si$, 90), 73 ($Me_3Si^+$, 100). The high resolution mass spectrum gave the value m/z of the molecular ion of 430.2925 (calculated for $C_{22}H_{46}O_4Si$ 430.2935).

**dl XLI(g):** The $^1H$ NMR spectrum ($CDCl_3$) of the product had characteristic shifts (δ) at 0.14 (s, 18, $Me_3Si$), 0.9 (m, 6, $CH_3$), 1.3 (m, 12, $(CH_2)_3$), 1.6 (m, 4, $OCH_2\underline{CH_2}$), 2.26 (s, 2, $Me_3SiCH$) 4.02 (m, 4, $OCH_2$). The $^{13}C$ NMR spectrum ($CDCl_3$) of the product had characteristic shifts (δ) at -0.75, 14.03, 22.58, 25.74, 28.72, 31.47, 34.78, 64.32, 174.67. The IR spectrum ($CCl_4$) of the product had a characteristic absorption of 1725 cm $^{1}$. The EIMS m/z of the product had characteristic peaks at 430 ($M^+$, 9), 415 ($M^+$ - $CH_3$, 11), 402 ($M^+$ - CO), 387 ($M^+$ - $CH_3$, CO, 13), 345 ($M^+$ - $C_6H_{13}$, 20), 329 ($M^+$ -$C_6H_{13}O$, 18), 301 ($M^+$ - $C_6H_{13}O$, CO, 29), 245 ($M^+$ -$CH_3$, $2C_6H_{13}$, 92), 201 (25), 171 (97), 147 (60), 129 (27), 99, (27), 73 ($Me_3Si^+$, 100). The high resolution mass spectrum gave the value m/z of the molecular ion of 430.2916 (calculated for $C_{22}H_{46}O_4Si_2$ 430.2935).

**dl- and meso-Di-n-dodecyl succinates XLI(h)**

**n-$C_{12}H_{25}OSiMe_3$ XLVI**

To 3 mL hexane containing 1-dodecanol (0.0430 g, 0.230 mmol) in a 5 mL flask under argon was added 1.6 M n-BuLi (72 mL, 0.115 mmol) in hexane with stirring. Bisketene **XI** (26.1 mg, 0.115 mml) in 1 mL hexane was added dropwise and the solution was stirred for 30 min at 25 °C. Then 5 mL hexane was added and the mixture was washed twice with water, dried over anhydrous $MgSO_4$, and evaporated to give 0.0484 g of yellow liquid, which was shown by $^1H$ NMR analysis to be a 1/3/1 mixture of trimethylsilyl n-dodecyl ether **XLVI** and the stereoisomeric esters **XLI(h)**, which were separated by radial chromatography (4% EtOAc).

**XLVI:** The $^1H$ NMR spectrum ($CDCl_3$) of the product had characteristic shifts (δ) at 0.11 (s, 9, $Me_3Si$),

0.9 (m, 3, $CH_3$), 1.3 (m), 1.6 (m), 3.57 (t, 2, $CH_2O$). The EIMS m/z of the product had characteristic peaks at 258 ($M^+$), 243 ($M^+$ - $CH_3$, 100) 103 (27), 97 (37), 89 ($Me_3SiO^+$, 17), 73 ($Me_3Si^+$, 36). The high resolution mass spectrum gave the value m/z of the ion ($M^+$ - $CH_3$) of 243.2143 (calculated for $C_{14}H_{31}OSi$ 243.2144).

**meso XLI(h):** The $^1H$ NMR spectrum ($CDCl_3$) of the product had characteristic shifts (δ) at 0.07 (s, 18, $Me_3Si$), 0.9 (m), 1.6 (m), 2.61 (s, 2, $Me_3SiCH$), 4.02 (m, 4, $OCH_2$). The $^{13}C$ NMR spectrum ($CDCl_3$) of the product had characteristic shifts (δ) at -1.60, 14.10, 22.68, 26.08, 28.63, 29.24, 29.33, 29.56, 29.62,31.91, 35.44, 64.29, 174.81 (peaks overlap between 29.24 and 29.62). The IR spectrum ($CCl_4$) of the product had a characteristic absorption of 1711 cm 1. The EIMS m/z of the product had characteristic peaks at 598 ($M^+$, 33), 55 ($M^+$ -$CH_3$, CO, 9), 429 ($M^+$ - $C_{12}H_{25}$, 22), 385 ($M^+$ -$C_{13}H_{25}CO_2$, 26), 340 ($M^+$ - $Me_3Si$, $C_{12}H_{25}O$, 24), 261 (92), 245 (87), 171 (89), 147 (34), 73 ($Me_3Si^+$, 100). The high resolution mass spectrum gave the value m/z of the molecular ion of 598.4788 (calculated for $C_{34}H_{70}O_4Si$ 598.4813).

**dl XLI(h):** The $^1H$ NMR spectrum ($CDCl_3$) of the product had characteristic shifts (δ) at 0.13 (s, 18, two $Me_3Si$), 0.9 (m), 1.3 (m), 1.6 (m), 2.26 (s, 2, $Me_3SiCH$), 4.0 (m, 4, $OCH_2$). The $^{13}C$ NMR spectrum ($CDCl_3$) of the product had characteristic shifts (δ) at -0.81, 14.10, 22.68, 26.04, 28.73, 29.25, 29.34, 29.53, 29.63, 31.91, 34.75, 64.32, 174.73 (peaks overlap between δ 29.25 and 29.63). The IR spectrum ($CCl_4$) of the product had a characteristic absorption of 1725 cm 1. The EIMS m/z of the product had characteristic peaks at 598 ($M^+$, 7), 429 ($M^+$ - $C_{12}H_{25}$, 5), 385 ($M^+$ -$C_{13}H_{25}CO_2$, 7), 340 ($M^+$ - $Me_3Si$, $C_{12}H_{25}O$, 7), 261 (53), 245 (58), 245 (58), 171 (70), 147 (30), 85 (24), 73 (100).

**threo- and erythro- methyl tert-butyl succinate (XLIII).**

To bisketene **XI** (21.9 mg, 0.097 mmol) in a 10 mL flask cooled in an ice bath was added cold MeOH (1.5 mL, 37 mmol,

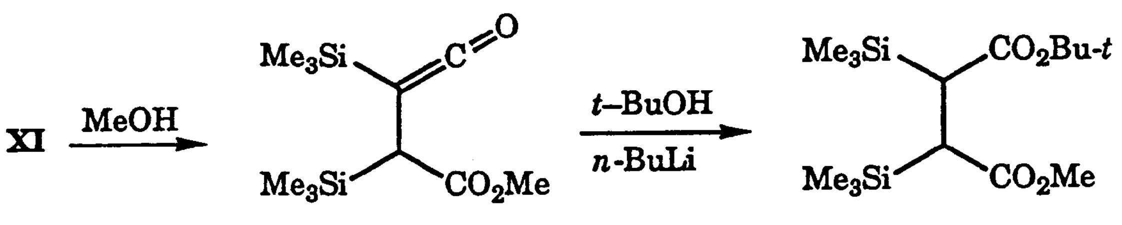


XLII XLIII

dried with 3A molecular sieves). After 7 min the solvent was removed under reduced pressure to give monoketene **XLII** (20.5 mg) as a yellowish oil. The $^1H$ NMR spectrum ($CDCl_3$) of the product had characteristic shifts (δ) at 0.14 (s, 9, $Me_3Si$), 0.15 (s, 9, $Me_3Si$).

A 2-necked 25 mL round bottom flask fitted with two septa and a magnetic stirrer was evacuated under argon for 5 min, and t-BuOH (1 mL, 0.011 mol) in 4 mL hexane was added followed by 1.6 M n-BuLi (0.4 mL, 0.026 mol) solution in hexane with stirring. A sample of **XLII** (0.0433 g) in 1.5 mL hexane was then added dropwise with stirring and the solution was stirred for 67 h at 25 °C. Then 10 mL of water was added and the mixture was extracted with hexane, dried over anhydrous $MgSO_4$, and evaporated to give 0.0342 g of colorless liquid. Radial chromatography (4% EtOAc in hexane) gave the stereoisomeric esters **XLIII** as colorless liquids identified by their spectra properties:

**erythro XLIII:** ($R_f$ = 0.3) The $^1H$ NMR spectrum ($CDCl_3$) of the product had characteristic shifts (δ) 0.081 (s, 9, $Me_3Si$), 0.099 (s, 9, $Me_3Si$), 1.456 (s, 9, t-BuO), 2.519 and 2.594 (each d, 1, J=11.6 Hz, $Me_3SiCH$), 3.600 (s, 3, MeO). The $^{13}C$ NMR spectrum ($CDCl_3$) of the product had characteristic shifts (δ) at - 1.54, -1.12, 28.39, 35.12, 36.55, 50.90, 80.17, 173.87, 175.29. The IR spectrum (film) of the product had a characteristic absorption of 1710 cm 1. The EIMS m/z of the product had characteristic peaks at 332 ($M^+$), 317 ($M^+$ -$CH_3$), 302, 289 ($M^+$ -$CH_3$ - CO), 275 ($M^+$ -t-Bu), 171, 73. The high resolution mass spectrum gave the value m/z of the molecular ion of 332.1849 (calculated. for $C_{15}H_{32}O_4Si_2$ 332.1839).

**threo XLIII :** The $^1H$ NMR spectrum ($CDCl_3$) of the product had characteristic shifts (δ) at 0.132 (s, 9, $Me_3Si$), 0.144 (s, 9, $Me_3Si$), 1.443 (s, 9, t-BuO), 2.178 and 2.224 (each d, J=6.1 Hz, $Me_3SiCH$), 3.644 (s, 3, MeO). The $^{13}C$ NMR spectrum ($CDCl_3$) of the product had characteristic shifts (δ) at -0.82, - 0.76, 28.23, 34.77, 35.62, 51.02, 79.88, 173.68, 175.20. The IR spectrum (film) of the product had a characteristic absorption of 1721 cm 1. The EIMS m/z of the product had characteristic peaks at 332 ($M^+$), 317 ($M^+$ $-CH_3$), 302, 289 ($M^+$ $-CH_3$ - CO), 275 ($M^+$ -t-Bu), 171, 73.

**Di-(1,2:3,4-diisopropylidene-D-galacto-6-pyranosyl) succinate**

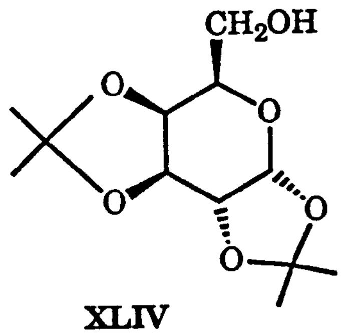

XLIV

To the sugar 1,2:3,4-diisopropylidene-D-galactopyranose **XLIV** ((102.5 mg, 0.394 mmol) in a 5-mL flask was added 4 mL of pentane. Only part of the sugar dissolved. To it was added 10 µL of a 2M pentane solution of n-BuLi. The mixture was combined with 0.5 mL of a pentane solution of bisketene **XI** (44.0 mg, 0.195 mmol) and stirred for three days. The pentane was evaporated and the residue was analyzed by the $^1H$ NMR spectrum ($CDCl_3$) at 400 MHz, which showed complete consumption of bisketene and signals due to the succinate **XLIi** with characteristic shifts (δ) at 0.07 (d, $Me_3Si$ of one isomer), 0.13 (d, $Me_3Si$ for the other isomer), 1.3-1.5 (several peaks,$Me_2C$), 2.26 (d, $Me_3SiCH$ of one isomer), 2.64 (d, $Me_3SiCH$ of the other isomer), 3.9-4.3 (m), 4.59 (m), 5.52 (m).

EXAMPLE 12

This example illustrates experiments undertaken to form compounds using the bisketene **XI**.

**Reaction between bisketene XI and 1,6 hexanediol.**

METHOD A: To 1.5 mL of pentane was added 20.3 mg of 1,6-hexanediol (0.172) mmol). 1.5 mL of anhydrous $MeOCH_2CH_2OMe$ (distilled over sodium/benzophenone) was then added to form a homogeneous solution, to which was added 20 µl of 2M pentane solution of N-BuLi (0.04 mmol). The resulting heterogeneous mixture was combined with about 40 mg of bisketene **XI** (0.17mmol). The mixture was maintained at room temperature and later turned brown overnight. The progress of the reaction was monitored with the $^1H$ NMR spectrum at intervals. At both t=17h and 41h, the $^1H$ NMR spectrum showed a sharp singlet at 1.954 ppm, two singlets at 2.6254 and 2.608 ppm and a broad multiplet at 4.1 ppm. The solvents in the reaction mixture were evaporated and the residue was retained uncapped for three days. Later $^1H$ NMR spectrum analysis of the residue showed that the singlet at 1.954 ppm disappeared, indicating the presence of a polymer **V** formed by coupling of the alcohol and ketene groupings to form ester linkages.

METHOD B: In a separate experiment, 1,6-hexanediol (126.5 mg, 0.14 mmol) and MeONa (0.5 mg, a catalyst) were mixed with 2 mL of anhydrous ether and the bisketene **XI** (32 mg, 0.14 mmol). The mixture was maintained at room temperature for 4 days with occasional stirring. $^1H$ NMR spectrum analysis of the crude product showed signals due to esters. Gel permeation chromatography (GPC) analysis (refractive index detector) showed a peak at 5.84 min, corresponding to an Mn of 1440 (using the poly-THF standards), indicating the presence of a polyester **V** having alternating 1,6 hexanediol and bisketene residues.

**Reaction between bisketene XI and 1,4 butanediol.**

To 1,4 butanediol (39.5, 0.438 mmol) in 3 mL of anhydrous THF in a 10 mL round-bottomed flask, was added the cyclobutenedione **X** (99.0 mg, 0.438 mmol). The reactants were refluxed for 17 hours. Evaporation of the solvent under reduced pressure gave 138 mg of yellow-brownish gum. The $^1H$ NMR spectrum ($CDCl_3$) of the product had characteristic shifts (δ) at 4.05, 3.88, and 3.60 ($OCH_2$ groups) in addition to many other signals and with an Mn of 1090 indicating the presence of a polyester **V** by addition of the OH groups of the butanediol

to the ketenyl moieties.

**Reaction between bisketene XI and 1,2-ethanediol.**

1,2-Ethanediol (43.5 mg, 0.702 mmol) and the cyclobutenedione **X** (158.6 mg, 0.702 mmol) in 10 mL of anhydrous THF were refluxed for 42 hours. Evaporation of the solvent gave a brownish oil. A crude product was obtained with an Mn of 1380, indicating the presence of a polyester **V**.

**Reaction of bisketene XI and poly-THF in the presence of n-BuLi**

Poly THF (Aldrich, MW=1000, 89.3 mg, 0.0893 mmol) and toluene (0.4mL) were placed in a 1 mL flask. To the mixture was added 10 µl of 2M pentane solution of n-BuLi (0.02 mmol) with a syringe. The mixture was sonicated at room temperature for one minute and then heated at 95° C for 8 minutes to facilitate formation of the corresponding lithium alkoxide. To the mixture was added the cyclobutenedione **X** (21 mg, 0.088 mmol). The mixture was heated at 95°C for 20 hours and then at 110°C for 24 hours. Gel permeation chromatography analysis showed an Mp of 45,880, indicating the presence of a polymer **XLVII** of poly(THF) connected by polyester bonds to residues of bisketene **XI**.

$$[OCH_2(CH_2OCH_2)_nCHR^1CHR^2CO]_m \qquad \textbf{XLVII}$$

**Reaction of bisketene XI with 1,2-ethanediol**

To ethanediol (3.3 µl, 0.048 mmol) in 0.5 mL THF and 1.6M n-BuLi (15 µl, 0.048 mmol) in hexane mixture was added bisketene **XI** (21.6 mg, 0.095 mmol) in 0.5 mL THF. The reaction was stirred for 20 hours at 25°C. After drying and evaporation of the ether, the $^1H$ NMR spectrum analysis of the brown liquid shown singlets at δ 0.125, 0.151, 0.156, 0.161, and multiplets at δ 3.8 and 4.2. IR (film) showed peaks at 1665, 1730, 1786, 1814, 1866 and a strong peak at 2090 $cm^{-1}$. LRMS (chemical ionization, isobutane) analysis gave a peak at m/z 514 while no peak was found at m/z 288, which suggests the presence of a new bistketene of the structure **VII** (where n=1, $R^1$, $R^2$ = $Me_3Si$).

**Reaction of bisketene XI with 1,4 cyclohexanediol.**

A mixture was formed with a molar ratio of 1:2:1 of bisketene:diol:n-BuLi. The mixture was stirred at 25°C for 6 hours. The $^1H$ NMR spectrum ($CDCl_3$) showed singlets at δ 0.069, 0.150 and multiplets at δ 4.1 consistent with the formation of polymer of type **V**.

**Reaction of bisketene XI with 1,4-phenylenediamine.**

Polyamides were prepared by reaction of bisketene **XI** generated in situ by heating cyclobutenedione **X** at 155° C with the diamine 1,4-phenylenediamine in THF. A solution of **XI** (320 mg, 1.42 mmol) and 1,4-phenylenediamene (157 mg, 1.42 mmol) in 5 mL anhydrous THF under argon was heated at 155°C for 18 hours. Evaporation of the solvent left a fibrous solid that was insoluble in methanol. This product is believed to be a polyamide **VI**.

**Reaction of bisketene XI with 1,6-diaminohexane.**

Polyamides were prepared by reaction of bisketene **XI** generated in situ by heating cyclobutenedione **X** at 155 C with the diamine 1,6-diaminohexane in THF to form the polyamide **VI**. A solution of cyclobutenedione **X** (300 mg, 1.33 mmol) and 1,6-diaminohexane (154 mg, 1.33 mmol) in 5 mL anhydrous THF under argon was heated at 155°C for 16 hours to give a yellow solid product.

**N,N'-Dimethyl-N,N'-diphenyl-2-trimethylsilyl-3-phenylsuccinamide**

The diketone **XVII** (23.2 mg, 0.1901 mol) in 0.5 mL $CDCl_3$ in an NMR tube was photolyzed for 1.5 hours with 350 nm light at room temperature. N-Methylaniline (10µL, 0.92

$$PhN(Me)OCHR^1CHR^2CON(Me)Ph \ \textbf{XLVIII}$$
$$R^1= Me_3Si \ R^2= Ph$$

mmol) was added with shaking and the sample was left at 0°C for 10 hours. The solvent was evaporated and the residue purified by radial chromatography to give **XLVIII** (13.5 mg, 0.0304 mmol, 30%) with a melting point

of 121.5-123.5 °C. The $^1H$ NMR spectrum (acetone-$d_6$) of the product had characteristic shifts at d 0.03 (s, 9, $Me_3Si$), 2.60 (d,1,J=11.4 Hz, CHTMS), 2.83 and 3.08 (each s, 3, MeN), 4.04 (d, 1, J= 11.4 Hz, PhCH), 6.20-7.50 (m, 15, 3 Ph). The $^{13}C$ NMR spectrum (acetone-$d_6$) of the product had characteristic shifts (δ) at -0.31, 36.77, 37.83, 41.97, 51.20, 127.45, 128.10, 128.17, 128.51, 128.65, 129.37, 129.50, 130.01, 130.07, 142.07, 144.24, 145.17, 172.63 and 173.42. The IR spectrum (film) of the product had a characteristic absorption of 1651, 1635 $cm^{-1}$ (C=O). The EIMS m/z of the product had characteristic peaks at 444 ($M^+$, 3), 429 ($M^+$, - $CH_3$, 11), 338 ($M^+$ -MeNPh, 100), 310 ($M^+$ -CONMePh, 32), 107 ($PhNHMe^+$, 20), 106 ($PhNMe^+$,21). The high resolution mass spectrum gave the value m/z of the product of 444.2235(cf. calculated for $C_{27}H_{32}SiN_2O_2$ 444.2233).

## Claims

1. A 1,2-bisketene of Formula I:

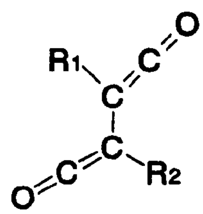

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group.

2. A compound as defined in claim 1 wherein each of $R^3$, $R^4$ and $R^5$ comprise $C_{2-6}$ linear or branched alkyl groups.

3. A compound as defined in claim 1 or 2 wherein $R^1$ is trimethylsilyl, t-butyldimethylsilyl, triisopropylsilyl or triphenylsilyl.

4. A compound as defined in any one of claims 1 to 3 wherein $R^2$ is selected from the group consisting of trimethylsilyl, t-butyldimethylsilyl, phenyl, methyl, hydrogen, triisopropylsilyl and triphenylsilyl.

5. A process for producing a 1,2-bisketene of Formula I:

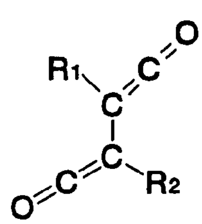

I

wherein:

$R^1$ is $SiR^3R^4R^5$;

$R^2$ is selected from the group comprising $SiR^3R^4R^5$, hydrogen, methyl, a substituted or unsubsti-

tuted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group;

the process comprising the step of subjecting a compound of Formula II:

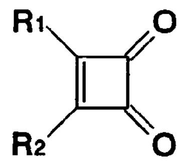

II

wherein $R^1$ and $R^2$ are as defined hereinbefore, to intramolecular ring opening to produce the 1,2-bisketene of Formula I.

6. A process as defined in claim 5 wherein said step of intramolecular ring opening includes subjecting the compound of Formula II to electromagnetic radiation having a wavelength from about 200 to about 400 nanometers.

7. A process as defined in claim 6 wherein said step of intramolecular ring opening is carried out in an inert solvent, at a maximum temperature of 50°C and in the absence of oxygen.

8. A process as defined in claim 5 wherein the step of intramolecular ring opening includes subjecting the compound of Formula II to heat in a range from about 25°C to 250°C.

9. A process for forming a succinate ester, comprising the steps of:

forming a reaction mixture including a 1,2-bisketene of Formula I:

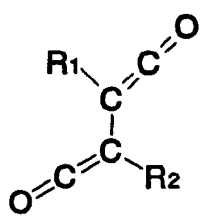

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen;

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group;

adding to said reaction mixture an alkoxide of an alcohol for reaction with said bisketene to form an enolate;

adding to said reaction mixture said alcohol for reaction with said enolate to form a monoketene;

adding to said reaction mixture said alkoxide to react with said monoketene to form an enolate; and

adding to said reaction mixture said alcohol for reaction with said enolate to form a succinate of Formula III:

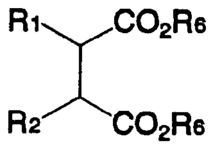

III

wherein $R^1$ and $R^2$ are defined as hereinbefore and $R^6$ is selected from the group comprising methyl, a substituted or unsubstituted $C_{2-20}$ linear or branched alkyl group, a substituted or unsubstituted $C_{6-20}$ aryl group and a substituted or unsubstituted $C_{5-20}$ cycloalkyl group.

**10.** A process as defined in claim 9, wherein said step of adding to said reaction mixture in alkoxide of an alcohol includes the steps of:

adding to said reaction mixture said alcohol and a reagent selected from the group consisting $R^7Na$, $R^7Li$ and $R^7K$ where $R^7$ is selected from the group comprising methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group;

selecting conditions to cause said reagent to react with said alcohol to form said alkoxide.

**11.** A process as defined in claim 10 wherein $R^6$ includes methyl, ethyl, n-hexyl, t-butyl and iso-propyl groups.

**12.** A process to form a monoketene useful for the production of polymers, comprising the steps of:

forming a reaction mixture including a bisketene of Formula I:

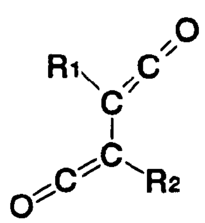

I

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group and substituted or unsubstituted $C_{7-20}$ aralkyl group and substituted or unsubstituted a $C_{5-20}$ cycloalkyl group;

adding to said reaction mixture a compound having a first reactive group; and

subjecting said reaction mixture to conditions to cause said bisketene to react with said first reactive group to yield said monoketene.

**13.** A process as defined in claim 12, wherein said reaction mixture includes a compound having a second reactive group, further comprising the step of selecting conditions to cause said monoketene to react with said second reactive group.

**14.** A process as defined in claim 13, further comprising the steps of:

removing said compound with said first reactive group from said reaction mixture; and

adding to said reaction mixture another compound including said second reactive group.

**15.** A process as defined in claim 13, wherein said compound is an alcohol or an amine.

**16.** A process as defined in claim 13, wherein said compound with said second reactive group is a bisketene, wherein said step of selecting conditions to cause said monoketene to react with second reactive group yields a derivatized bisketene.

**17.** A process as defined in claim 13, wherein said compound includes both said first and second reactive groups.

**18.** A process as defined in claim 17, wherein said compound is a polymer selected from the group comprising latex, acrylic and carbohydrate, wherein said bisketene serves as a chain extender.

**19.** A monoketene of Formula XXXV:

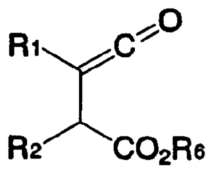

XXXV

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group;

$R^6$ is selected from the group comprising methyl, a substituted or unsubstituted $C_{2-20}$ linear or branched alkyl group, a substituted or unsubstituted $C_{6-20}$ aryl group and a substituted or unsubstituted $C_{5-20}$ cycloalkyl group.

**20.** A monoketene as defined in claim 19, wherein each of $R^3$, $R^4$ and $R^5$ comprise $C_{2-6}$ linear or branched alkyl groups.

**21.** A monoketene as defined in claim 20, wherein $R^1$ is trimethylsilyl, t-butyldimethylsilyl, triisopropylsilyl or triphenylsilyl.

**22.** A monoketene as defined in claim 19, wherein $R^6$ includes methyl, ethyl, n-hexyl, t-butyl and iso-propyl groups.

**23.** A succinate ester of Formula III:

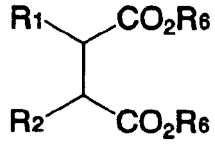

III

wherein:

$R^1$ is $SiR^3R^4R^5$;

$R^2$ is selected from the group comprising $SiR^3R^4R^5$, hydrogen, methyl, a substituted or unsubstituted $C_{2-20}$ linear or branched alkyl group, a substituted or unsubstituted $C_{6-20}$ aryl group, a substituted or unsubstituted $C_{5-20}$ cycloalkyl group and a halogen; and

each of $R^3$, $R^4$ and $R^5$ are the same or different and are selected from the group comprising hydrogen, methyl, a substituted or unsubstituted $C_{2-20}$ linear or branched alkyl group, a substituted or unsubstituted $C_{6-20}$ aryl group, a substituted or unsubstituted $C_{7-20}$ aralkyl group and substituted or unsubstituted a $C_{5-20}$ cycloalkyl group;

$R^6$ is selected from the group comprising methyl, a substituted or unsubstituted $C_{2-20}$ linear or branched alkyl group, a substituted or unsubstituted $C_{6-20}$ aryl group and a substituted or unsubstituted $C_{5-20}$ cycloalkyl group.

**24.** A succinate ester as defined in claim 23, wherein each of $R^3$, $R^4$ and $R^5$ comprise $C_{2-6}$ linear or branched alkyl groups.

**25.** A succinate ester as defined in claim 24, wherein $R^1$ is trimethylsilyl, t-butyldimethylsilyl, triisopropylsilyl or triphenylsilyl.

European Patent Office

# EUROPEAN SEARCH REPORT

Application Number
EP 94 30 7926

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | J. AM. CHEM. SOC. (JACSAT,00027863);92; VOL.114 (27); PP.10980-1, UNIV. TORONTO;DEP. CHEM.; TORONTO; M5S 1A1; ON; CAN. (CA) Zhao D C et al 'A stable and persistent bisketene: 2,3-bis(trimethylsilyl)-1,3-butadiene-1,4-dione' * the whole document * --- | 1-25 | C07F7/08 C07H23/00 //C08G77/60 |
| P,X | J. AM. CHEM. SOC. (JACSAT,00027863);93; VOL.115 (22); PP.10097-103, UNIV. TORONTO;DEP. CHEM.; TORONTO; M5S 1A1; ON; CAN. (CA) Zhao D C et al 'Preparation and reactivity of persistent and stable silyl-substituted bisketenes' * the whole document * | 1-25 | |
| X | & ADVANCE ACS ABSTRACTS, 1 October 1993 --- | 1-25 | |
| X | J. ORG. CHEM. (JOCEAH,00223263);92; VOL.57 (14); PP.3994-4000, UNIV. BASEL;DEP. CHEM.; BASEL; CH-4056; SWITZ. (CH) Kopping B et al 'Tris(trimethylsilyl)silane: an efficient hydrosilylating agent of alkenes and alkynes' * compound 15a * --- | 23-25 | TECHNICAL FIELDS SEARCHED (Int.Cl.6) C07F C07H |
| X | J. ORGANOMET. CHEM. (JORCAI);74; VOL.77 (2); PP.167-76, UNIV. BORDEAUX I;LAB. CHIM. ORG.; TALENCE; FR. Picard J P et al 'Action of.alpha.-ethylenic esters on the trimethylsilicon chloride/magnesium/HMPT[hexamethylphosphoric triamide] system' * page 170 * --- -/-- | 23-25 | |

The present search report has been drawn up for all claims

| Place of search | Date of completion of the search | Examiner |
|---|---|---|
| THE HAGUE | 1 February 1995 | Rinkel, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 94 30 7926

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 080, no. 9, 4 March 1974, Columbus, Ohio, US; abstract no. 048086, BREVNOVA T N ET AL 'Thermal decomposition of tert-butyl.beta.-(triethylsilyl)perpropionat' * abstract * & ZH. OBSHCH. KHIM. (ZOKHA4);73; VOL.43 (10); PP.2228-32, INST. KHIM.;GORKI; USSR --- | 23-25 | |
| P,X | J. AM. CHEM. SOC. (JACSAT,00027863);94; VOL.116 (6); PP.2625-6, UNIVERSITY OF TORONTO;DEPARTMENT OF CHEMISTRY; TORONTO; M5S 1A1; ON; CAN. (CA) Allen A D et al 'Formation and Reactivity of Unsymmetrical Bis(ketenes): 2-Phenyl- and 2-Methyl-3-(trimethylsilyl)-1,3-butadiene-1,4-diones' * the whole document * --- | 1-25 | |
| P,X | J. AM. CHEM. SOC. (JACSAT,00027863);94; VOL.116 (16); PP.7233-8, UNIVERSITY OF TORONTO;DEPARTMENT OF CHEMISTRY; TORONTO; M5S 1A1; ON; CAN. (CA) McAllister M A et al 'Ab Initio Calculations of the Ring Opening of Cyclobutene-1,2-diones and Conformational Properties of the Product 1,3-Butadiene-1,4-diones (Bisketenes)' * the whole document * --- | 1-25 | TECHNICAL FIELDS SEARCHED (Int.Cl.6) |
| P,X | TETRAHEDRON LETT. (TELEAY,00404039);94; VOL.35 (26); PP.4565-8, UNIV. LAUSANNE;INST. CHIM. ORG.; LAUSANNE; 1005; SWITZ. (CH) Landais Y et al 'Asymmetric metal carbene insertion into the Si-H bond' * the whole document * ----- | 23-25 | |

The present search report has been drawn up for all claims

| Place of search | Date of completion of the search | Examiner |
|---|---|---|
| THE HAGUE | 1 February 1995 | Rinkel, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)